(19) Europäisches Patentamt / European Patent Office / Office européen des brevets

(11) EP 4 787 379 A1

(12) EUROPEAN PATENT APPLICATION

(43) Date of publication:
05.08.2026 Bulletin 2026/32

(21) Application number: 26150173.8

(22) Date of filing: 16.01.2018

(51) International Patent Classification (IPC):
G16B 20/20 (2019.01)

(52) Cooperative Patent Classification (CPC):
G16B 20/20; C12Q 1/6886; G16B 30/10

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 17.01.2017 US 201762447382 P

(62) Document number(s) of the earlier application(s) in accordance with Art. 76 EPC:
18702868.3 / 3 571 613

(71) Applicant: Illumina, Inc.
San Diego, CA 92122 (US)

(72) Inventors:
• SNEDECOR, June
San Diego, 92122 (US)
• CHUANG, Han-yu
San Diego, 92122 (US)
• BERRY, Gwenn
San Diego, 92122 (US)
• CHEN, Xiao
San Diego, 92122 (US)

(74) Representative: Witte, Weller & Partner
Patentanwälte mbB
Postfach 10 54 62
70047 Stuttgart (DE)

Remarks:
• This application was filed on 05-01-2026 as a divisional application to the application mentioned under INID code 62.
• Claims filed after the date of filing of the application / after the date of receipt of the divisional application (Rule 68(4) EPC).

(54) ONCOGENIC SPLICE VARIANT DETERMINATION

(57) Presented herein are systems and methods for identifying splice variants. The techniques include determining one or more sample splice junctions from a plurality of RNA sequence reads from a single biological sample, retrieving a set of baseline splice junctions determined from a plurality of healthy RNA samples and comparing the one or more sample splice junctions to the set of baseline splice junctions to identify one or more filtered sample splice junctions comprising sample splice junctions that do not overlap with the baseline splice junctions, wherein the one or more filtered sample splice junctions are candidate oncogenic events.

FIG. 8

EP 4 787 379 A1

## Description

INCORPORATION BY REFERENCE TO PRIORITY APPLICATIONS

The present application claims the benefit of priority to U.S. Provisional Appl. No. 62/447,382, filed January 17, 2017, which is hereby incorporated by reference.

BACKGROUND

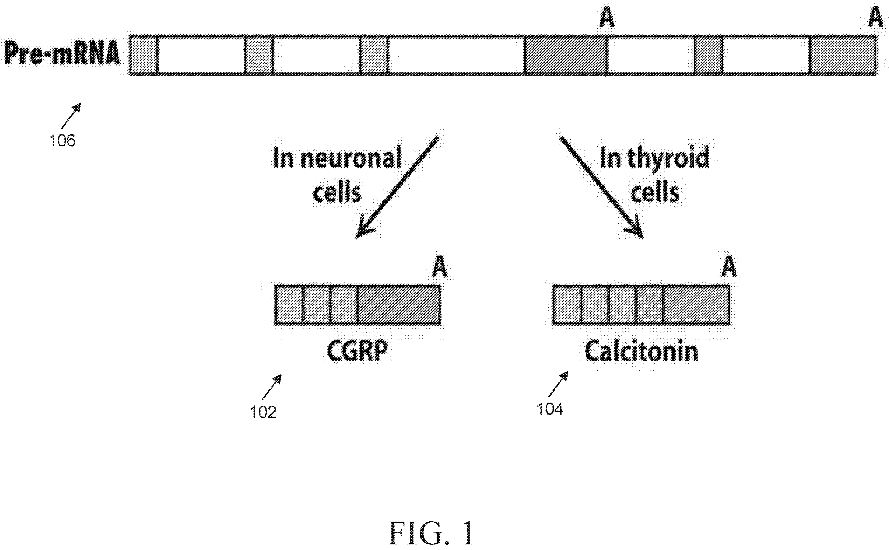

A splice variant is a single variation of a gene transcript. Many genes have multiple possible splice variants which allow for a single gene to encode multiple possible proteins depending on cell environment or function. Prior to being translated into a protein, an mRNA transcript is spliced to remove regions of the mRNA transcript that are not to be encoded in a protein sequence. As illustrated in FIG. 1, calcitonin gene-related peptide (CGRP) 102 and calcitonin 104 are produced by the same source gene transcript, expressed as precursor mRNA (pre-mRNA) 106, and are spliced differently depending on where the gene transcript is expressed. As a non-limiting example, the pre-mRNA 106 may be either spliced as CGRP 102 when present in neuronal cells, or spliced as calcitonin 104 when present in thyroid cells.

Traditionally, oncogenic splice variants may be determined from a patient by acquiring a set of non-tumor samples and a set of tumor samples. Then, each of the samples are sequenced and mapped to a reference (either DNA or RNA). Subsequently, whole splice transcripts are identified de-novo and expression differences between the normal (non-tumor) and abnormal (tumor) samples are evaluated based upon the splice transcript.

Traditional methods of determining oncogenic splice variants are not ideal due to requiring multiple samples. Also, running multiple samples for a single patient drastically increases both reagent and sequencing costs. For example, costs could at least be doubled if paired tumor/non-tumor samples are required.

SUMMARY

The Summary is provided to introduce a selection of concepts in a simplified form that are further described below in the Detailed Description. The Summary is not intended to identify key features or essential features of the claimed subject matter, nor is it intended to be used to limit the scope of the claimed subject matter.

One aspect of the disclosure provides various methods and systems for identifying splice variants. In one implementation, a method comprises: determining one or more sample splice junctions from a plurality of RNA sequence reads from a single biological sample; retrieving, a set of baseline splice junctions determined from a plurality of healthy RNA samples; comparing the one or more sample splice junctions to the set of baseline splice junctions; and identifying one or more filtered sample splice junctions, the filtered sample splice junctions comprising sample splice junctions that do not overlap with the baseline splice junctions, wherein the one or more filtered sample splice junctions are candidate oncogenic events.

Some embodiments further comprise outputting the list of candidate oncogenic events.

In some embodiments, the plurality of healthy RNA samples comprises healthy RNA samples taken from a cross section of one or more of: geographical regions, ages, genders, ethnic groups, tissue types, or sample preservation qualities type.

In some embodiments, the plurality of healthy RNA samples comprises samples from one or more tissue types selected from the group consisting of: lung, adrenal gland, bladder, breast, ovary, liver, prostate, skin, and spleen. In some embodiments, the plurality of healthy RNA samples comprises samples from donors across a range of ages.

In some embodiments, the baseline splice junctions from the plurality of healthy RNA samples are determined prior to the determining the sample junctions from the single sample.

In some embodiments, the plurality of healthy RNA samples for the base line splice junctions are not obtained from the same biological object as the single biological sample.

In some embodiments, the baseline junctions are from a same genomic region as the sample junctions.

In some embodiments, the single biological sample is from a tumor sample.

In some embodiments, the sample splice junctions and the baseline splice junctions are both determined using a common assay.

In some embodiments, determining the one or more sample junctions comprises: determining the plurality of RNA sequence reads from the single biological sample; retrieving, a DNA reference sequence aligned with the RNA sequence reads from the single biological sample; and determining one or more sample junctions as missing contiguous locations in the RNA read compared with the DNA reference.

In some embodiments, the filtered sample splice junctions do not overlap with third party junctions, the third party junctions determined from a splice graph that captures multiple alternate combinations of exons for a given gene.

In some embodiments, the set of baseline splice junctions are determined without determining a splice graph that captures multiple alternate combinations of exons for a given gene.

Some embodiments provide a system for identifying splice variants. The system includes a memory, at least one processor; and at least one non-transitory computer-readable medium containing instructions that, when executed by the at least one processor, cause the at least one processor to perform operations comprising determining one or more sample splice junctions from a plurality of RNA sequence reads from a single biological sample; retrieving, a set of baseline splice junctions determined from a plurality of healthy RNA samples; comparing the one or more sample splice junctions to the set of baseline splice junctions; and identifying one or more filtered sample splice junctions, the filtered sample splice junctions comprising sample splice junctions that do not overlap with the set of baseline splice junctions, wherein the filtered sample splice junctions are candidate oncogenic events.

As described herein, a variety of other features and advantages can be incorporated into the technologies as desired.

The invention includes the following aspects defined in the following clauses which form part of the present description, which, however, do not represent claims, in accordance with the decision J15/88 of the Legal Board of Appeal of the European Patent Office.

1. A system for identifying splice variants comprising:

   a memory;
   at least one processor; and
   at least one non-transitory computer readable medium containing instructions that, when executed by the at least one processor, cause the at least one processor to perform operations comprising:

      determining one or more sample splice junctions from a plurality of RNA sequence reads from a single biological sample;
      retrieving a set of baseline splice junctions, the set of baseline splice junctions determined from a plurality of healthy RNA samples;
      comparing the one or more sample splice junctions to the set of baseline splice junctions; and
      identifying one or more filtered sample splice junctions, the filtered sample splice junctions comprising sample splice junctions that do not overlap with the set of baseline splice junctions,
      wherein the filtered sample splice junctions are candidate oncogenic events.

2. The system of clause 1, further comprising outputting the list of candidate oncogenic events.

3. The system of clause 1 or clause 2, wherein the plurality of healthy RNA samples comprises healthy RNA samples taken from a cross section of one or more of: geographical regions, ages, genders, ethnic groups, tissue types, or sample preservation qualities.

4. The system of any one of clauses 1-3, wherein the plurality of healthy RNA samples comprises samples from one or more tissue types selected from the group consisting of: lung, adrenal gland, bladder, breast, ovary, liver, prostate, skin, and spleen.

5. The system of any one of clauses 1-4, wherein the plurality of healthy RNA samples comprises samples from donors across a range of ages.

6. The system of any one of clauses 1-5, wherein the baseline splice junctions from the plurality of healthy RNA samples are determined prior to the determining the sample junctions from the single sample.

7. The system of any one of clauses 1-6, wherein the plurality of healthy RNA samples for the baseline splice junctions are not obtained from the same biological object as the single biological sample.

8. The system of any one of clauses 1-7, wherein the baseline junctions are from a same genomic region as the sample junctions.

9. The system of any one of clauses 1-8, wherein the single biological sample is from a tumor sample.

10. The system of clause 9, wherein the plurality of healthy RNA samples are from non-tumor tissue.

11. The system of any one of clauses 1-10, wherein the sample splice junctions and the baseline splice junctions are both determined using a common assay.

12. The system of any one of clauses 1-11, wherein determining the one or more sample junctions comprises:

   determining the plurality of RNA sequence reads from the single biological sample;
   retrieving, a DNA reference sequence aligned with the RNA sequence reads from the single biological sample; and
   determining one or more sample junctions as missing contiguous locations in the RNA read compared with the DNA reference.

13. The system of any one of clauses 1-12, wherein the filtered sample splice junctions do not overlap with third party junctions, the third party junctions determined from a splice graph that captures multiple alternate combinations of exons for a given gene.

14. The system of any one of clauses 1-13, wherein the set of baseline splice junctions are determined without determining a splice graph that captures multiple alternate combinations of exons for a given gene.

15. A computer implemented method, comprising:

determining, using at least one processor, one or more sample splice junctions from a plurality of RNA sequence reads from a single biological sample;

retrieving, by the at least one processor from a memory, a set of baseline splice junctions determined from a plurality of healthy RNA samples;

comparing the one or more sample splice junctions to the set of baseline splice junctions; and

identifying, by the at least one processor, one or more filtered sample splice junctions, the filtered sample splice junctions comprising sample splice junctions that do not overlap with the baseline splice junctions,

wherein the one or more filtered sample splice junctions are candidate oncogenic events.

16. The method of clause 15, further comprising outputting the list of candidate oncogenic events.

17. The method of clause 15 or clause 16, further comprising:

determining, by the at least one processor, RNA reads from the single sample;

retrieving, by the at least one processor from the memory, a DNA reference aligned with the RNA reads from the single sample; and

determining, by the at least one processor, the sample junctions as missing contiguous locations in the RNA read compared with the DNA reference.

18. The method of any one of clauses 15-17, wherein the plurality of healthy RNA samples comprises healthy RNA samples taken from a cross section of one or more of: geographical regions, ages, genders, ethnic groups, tissue types, or sample preservation qualities.

19. The method of any one of clauses 15-18, wherein the plurality of healthy RNA samples for the baseline splice junctions are not obtained from the same biological object as the single biological sample.

20. The method of any one of clauses 15-19, wherein the filtered sample junctions do not overlap with third party junctions, the third party junctions determined from a splice graph that captures multiple alternate combinations of exons for a given gene.

## BRIEF DESCRIPTION OF THE DRAWINGS

FIG. 1 is a conceptual diagram illustrating exemplary features of a splice variant.

FIG. 2 is a block diagram illustrating an embodiment of an operating environment including splice variant determination.

FIG. 3 is a block diagram illustrating an embodiment of example components of a splice variant determination service utilized in accordance with the operating environment of FIG. 2.

FIG. 4 is a flow diagram illustrating an embodiment of junction analysis.

FIG. 5 is a flow diagram illustrating an embodiment of determining possible oncogenic junctions.

FIG. 6 is a flow diagram illustrating an embodiment of determining sample junctions.

FIG. 7 is a flow diagram illustrating an embodiment of determining baseline junctions.

FIG. 8 is a flow diagram illustrating an embodiment of determining filtered sample junctions.

FIG. 9 is a flow diagram illustrating an embodiment of verifying filtered sample junctions.

FIG. 10 is a flow diagram with accompanying conceptual illustration of an embodiment of determining possible oncogenic junctions.

FIG. 11 is a table illustrating experimental results from the embodiment of FIG. 10.

FIG. 12A and FIG. 12B are conceptual diagrams illustrating features of verifying filtered sample junctions.

## DETAILED DESCRIPTION

Generally described, the present disclosure corresponds to methods and systems for oncogenic splice variant determination via baseline analysis.

Splicing may often be disrupted in cancerous cells. Disruptions that cause splicing variations have been identified in many cancers, as described in Dvinge, H., & Bradley, R. K. (2015), "Widespread intron retention diversifies most cancer transcriptomes" Genome Medicine, 7(1), 45., incorporated herein by reference in its entirety. Additionally, pharmaceutical companies have identified the products of these variants as potential targets for drug therapies. The ability to identify patients who carry the affected variants may be important in studying the efficacy of drugs for cancer treatment.

There are a number of mutations on the DNA level that can lead to abnormal splicing in cancer (splice variants). Non-limiting examples may be found in Jung, H., Lee, D., Lee, J., Park, D., Kim, Y. J., Park, W.-Y., ... Lee, E. (2015), "Intron retention is a widespread mechanism of tumor-suppressor inactivation", Nature Genetics, 47(11), 1242-1248., incorporated herein by reference in its entirety.

The Cancer Genome Atlas (TCGA) (managed by the National Cancer Institute's Center for Cancer Genomics, headquartered in Rockville Maryland, USA, and the National Human Genome Research Institute, headquartered in Bethesda, Maryland, USA) has identified multiple mechanisms for mutations (splice variants), including at least the following: (1) direct splice site mutations; (2) mutations occurring within 30 base pairs (bp) of the last base of an exon; (3) changes to the transcript which do not occur near the affected exons but change where splicing happens; and (4) oncogenic changes not directly related to splicing (such as, but not limited to Myc mutations).

Therefore, it may be advantageous to identify splice variants by directly examining RNA rather than DNA for relevant changes due at least in part to the wide variety of mechanisms which can lead to disrupted splicing.

Furthermore, systems and methods in accordance with various embodiments described herein for oncogenic splice variant determination via baseline analysis determine possible oncogenic splice variants simply and without the drawbacks of traditional methodologies. As described above, the traditional methodologies of splice variant determination are more invasive, computationally intensive and costly due at least in part to employing multiple biopsies, or samples, from a patient. Rather, as described further below, a single sample of a tumor may be taken from a patient and compared with a baseline reference of healthy samples. This type of variant identification using a single tumor sample without a matched normal, healthy sample reduces the complexity of the analysis, focusing on verifiable abnormal events that are not expressed in normal, healthy samples.

Accordingly, oncogenic splice variant determination via baseline analysis focuses on relevant factors for splice variant determination, such as splice junction determination as described further below, and avoids the complex (and computational resource intensive) process of determining genomic expression de-novo. Stated another way, rather than building a splice graph of an entire transcript that captures in a single structure multiple (or all) alternate ways in which exons for a given gene may be assembled, splice variants may be evaluated on a junction level.

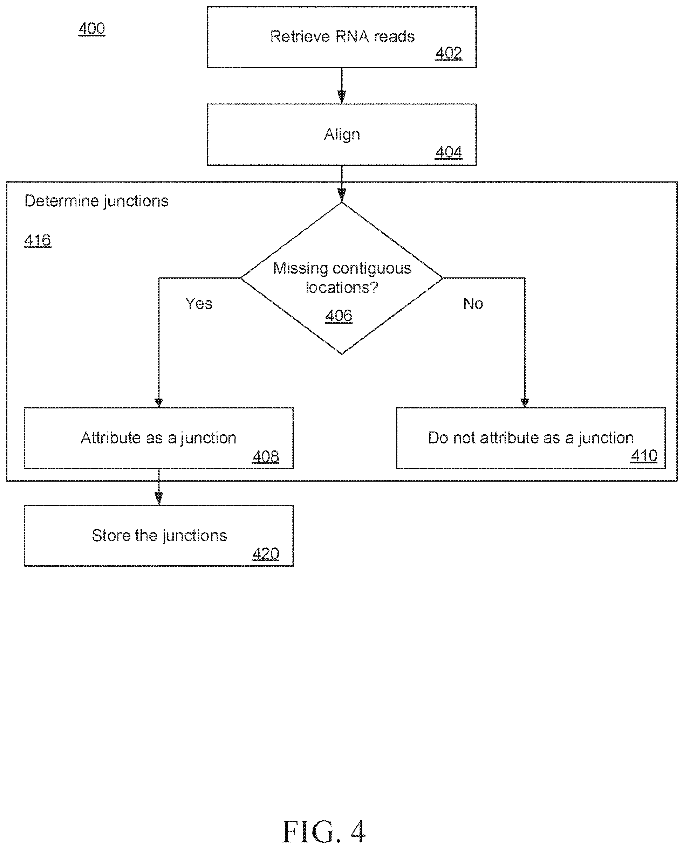

Splice junctions (also termed as junctions) define splice variants as coordinates on a DNA reference which do not appear in an RNA sequence, when aligned with the DNA reference. Junctions may be determined via an assay, which is a test for particular content (such as RNA for an RNA assay). Junctions will be discussed further below, at least in connection with FIG. 4. As a non-limiting example, for MET exon 14 skipping mutations in lung cancer, the junction between 13 and 15 may be determined to be a splice variant.

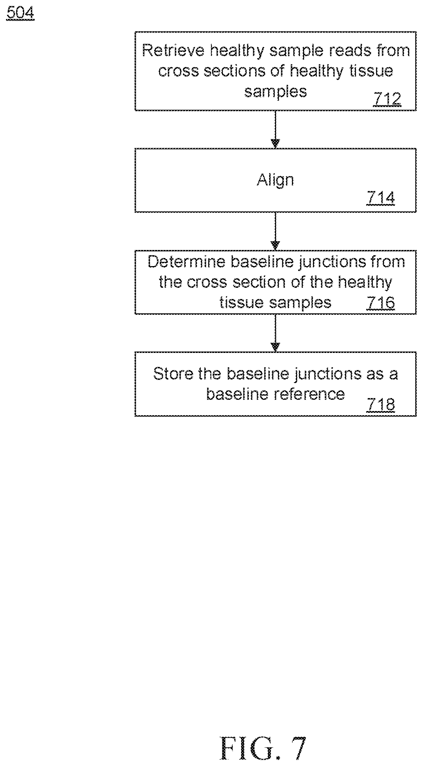

As introduced above, a baseline reference is a collection of a cross section of junctions from healthy, non-tumor samples. This baseline reference of junctions (or splice variants) observed in various cross sections of healthy, non-tumor tissues may be sequenced by a same RNA assay used to sequence a tumor sample under investigation. The baseline reference may be used to capture splicing events in normal physiology or caused by assay artifacts. The use of the baseline reference complements the limited curation of transcription isoforms in literature and reduces artifacts in formalin-fixed paraffin-embedded (FFPE) preservation or other systematic errors. Baseline analysis, or filtering by the baseline junctions of the baseline reference, captures novel splice junctions which are more likely to be associated with cancer. The baseline junctions of the baseline reference is discussed further below, at least in connection with FIG. 7.

However, it can be noted that these savings in computational resources may be balanced with difficulty in determining splice variants associated with cancer that are constitutively expressed in normal tissues, such as but not limited to certain variants for RPS6KB1 as described in Ben-Hur, V., Denichenko, P., Siegfried, Z., Maimon, A., Krainer, A., Davidson, B., & Karni, R. (2013), "S6K1 Alternative Splicing Modulates Its Oncogenic Activity and Regulates mTORC1", Cell Reports, 3(1), 103-115, incorporated herein by reference in its entirety. Nevertheless, as introduced above, oncogenic splice variant determination via baseline analysis features a number of advantages over traditional tools that may outweigh these difficulties.

## Overview of an Example Embodiment

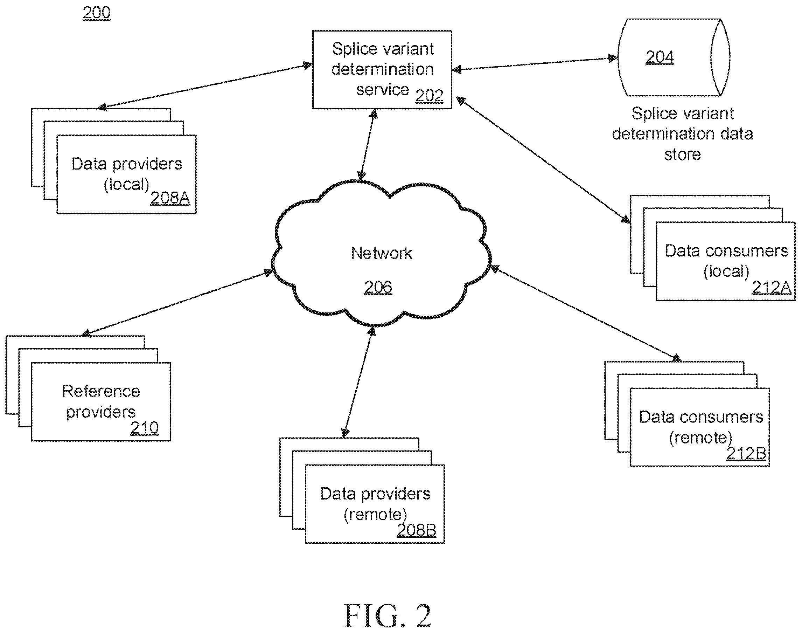

FIG. 2 illustrates an embodiment of a splice variant determination environment 200 that can implement the features described herein in the context of an example splice variant determination service 202. In some embodiments, the splice variant determination environment 200 includes the splice variant determination service 202, a splice variant determination data store 204, a network 206, local data providers 208A, remote data providers 208B, reference providers 210, local data consumers 212A, and remote data consumers 212B. In some embodiments, various components of the splice variant determination environment 200 are communicatively interconnected with one another via the network 206. The splice variant determination environment 200 may include different components, a greater or fewer number of components, and can be structured differently. For example, there can be more than one data store or other computing devices in connection with the splice variant determination service 202. As another example, components of the splice variant determination environment 200 may communicate with one another with or without the network 206.

The splice variant determination service 202 may correspond to any system capable of performing the processes described herein. The splice variant determination service 202 may be implemented by one or more computing devices. For example, the splice variant determination service 202 may be implemented by computing devices that include one or more processors to execute one or more instructions stored in memory, and communication devices to transmit and receive data over the network 206. In some embodiments, the splice variant determination service is implemented on one or more backend servers capable of communicating over a network. In other embodiments, the splice variant determination service 202 is implemented by one or more virtual machines in a hosted computing environment (e.g., a "cloud computing environment"). The hosted computing environment may include one or more provisioned and released computing resources, which computing resources may include computing, networking, and/or storage devices.

In one aspect, the splice variant determination service 202 can implement one or more applications that perform, individually or in combination, the splice variant determination functions described herein, including determining sample junctions, determining baseline junctions, determining a baseline reference, determining filtered sample junctions, determining RNA reads from tissue, removing junction overlap, verifying filtered sample junctions, determining sufficient overlap count, etc. These splice variant determination functions may be performed at different times and by different aspects of the splice variant determination service, such as (but not limited to) when the splice variant determination services does not determine baseline junctions of the baseline reference contemporaneously with determining sample filtered junctions or sample junctions, but rather initially determines and stores baseline junctions (collected as a baseline reference) and then retrieves the stored baseline junctions when determining sample filtered junctions. In another aspect, the splice variant determination service 202 may be configured to store or update baseline junctions, sample junctions at the splice variant determination data store 204. In some embodiments, the splice variant determination service may be associated with a network or network-based service provider or vendor.

In the illustrated embodiment, the splice variant determination service 202 may be communicatively connected to the splice variant determination data store 204. The splice variant determination data store 204 can generally include any repository, database, or information storage system that can store splice data and associated metadata. The splice data stored in the splice variant determination data store 204 can be baseline junctions of a baseline reference (including junctions determined from a cross section of healthy samples), tumor sample data from a single tumor sample, healthy sample data from a cross section of healthy or non-tumor samples, sample junctions from a single tumor sample, and/or filtered sample junctions processed in accordance with the oncogenic splice variant determination via baseline analysis. The splice data can be stored in various formats or data structures, such as lists, vectors, arrays, matrices, etc. Metadata can be associated with individual samples or junctions, or a collection of samples of junctions, for purposes of indicating their format, semantics, features, conditions, sources, data of creation, date of entry, date of annotation, date of processing, associated cross section (e.g., geographical region, age, gender, ethic group, FFPE artifacts, FFPE quality, homolog artifacts, polymerase read-through artifacts, non-oncological alternative splicing, tissue type), or the like. For example, metadata can link a sample junction from a single tumor sample determined via a common assay to baseline junctions determined via the common assay. Alternatively, or in addition, metadata may indicate a category or a position in a taxonomy associated with junctions in a collection of junctions (such as, but not limited to a baseline reference, a collection of baseline junctions, a collection of filtered sample junctions, or a collection of sample junctions).

The network 206 may include any suitable combination of networking hardware and protocols necessary to establish communications within the splice variant determination environment 200. For example, the network 206 may include private networks such as local area networks (LANs) or wide area networks (WANs), as well as public or private wired or wireless networks, satellite networks, cable networks, cellular networks, or the Internet. In such an embodiment, the network 206 may include hardware (e.g., modems, routers, switches, load balancers, proxy servers, etc.) and software (e.g., protocol stacks, accounting software, firewall/security software, etc.) implemented by hardware that establishes networking links within the splice variant determination environment 200. Additionally, the network 206 may implement one of various communication protocols for transmitting data between components of the splice variant determination environment 200.

The data providers 208A, 208B, may correspond to hosts of a local data provider 208A site (such as, but not limited to when a splice variant determination service 202 is on an instrument that also determines data from on-instrument RNA sequencing, or a device that stores such data from RNA sequencing) or a network or other remote data provider 208B site (such as, but not limited to when an instrument that determines data from RNA sequencing, or a device that stores such data from RNA sequencing, is remote from the splice variant determination service 202), or the like. Accordingly, the data providers 208A, 208B can be associated with any computing device(s) that can facilitate communications with the splice variant determination service 202 via, or in lieu of, the network 206. Such computing devices can generally include sequencing instruments, wireless mobile devices (e.g., smart phones, PDAs, tablets, wearable computing devices, or the like), servers, desktops, laptops, and computerized appliances, to name a few. Further, such computing devices can implement any type of software (such as a browser or a mobile application) that can facilitate the communications described above).

The data consumers 212A, 212B, may correspond to hosts of a local data consumer 208A site (such as, but not limited to when a splice variant determination service 202 is on an instrument on which other services or processes are dependent upon) or a network or other remote data provider 208B site (such as, but not limited to when a splice variant determination service 202 is on an instrument that is remote from the services or process are dependent upon it), or the like. The data consumers 212A, 212B may correspond to visitors to a clinical or research network site, scientists, doctors, bioinformaticians, engineers, or the like, and can be associated with any computing device(s) that can facilitate communication with the splice variant determination service 202 via, or in lieu of, the network 206. Such computing devices can generally include wireless mobile devices (e.g., smart phones, PDAs, tablets, wearable computing devices, or the like), servers, desktops, laptops, instruments, and computerized appliances, to name a few. Further, such computing devices can implement any type of software, (such as a browser or a mobile application) that can facilitate the communications described above.

The reference providers 210 may correspond to any entity that provides reference data related to the splice variant determination service 202, such as but not limited to reference genomes, DNA reference, RNA reference, splice graph of RNA transcripts, and third party junctions. In certain embodiments, the reference providers 210 provides the reference data to the splice variant determination service 202, and the splice variant determination service 202 stores the reference data locally in the splice variant determination data store 204. The reference providers 210 may correspond to a reference database network site, or the like, and can be associated with any computing device(s) that can facilitate communications with the splice variant determination service 202 via the network 206. Such computing devices can generally include wireless mobile devices (e.g., smart phones, PDAs, tablets, wearable computing devices, or the like), servers, desktops, laptops, instruments, and computerized appliances to name a few. Further, such computing devices can implement any type of software (such as a browser or a mobile application) that can facilitate the communications described above.

One skilled in the relevant art will appreciate that the components and configurations provided in FIG. 2 are illustrative in nature. Accordingly, additional or alternative components and/or configurations, including the additional components, systems, and subsystems for facilitating functions disclosed herein, may be utilized.

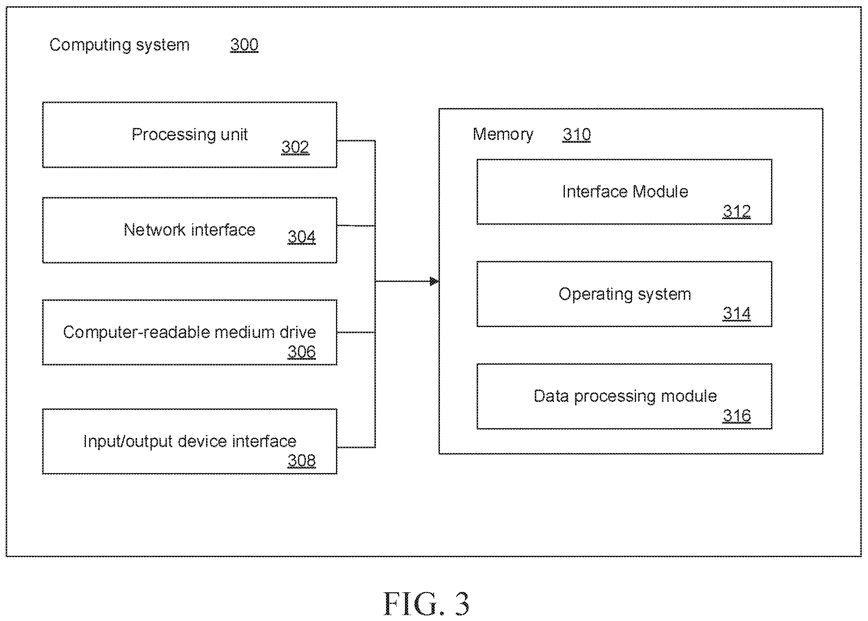

FIG. 3 is a block diagram illustrating an embodiment of example components of a variant calling service utilized in accordance with the operating environment of FIG. 2. The example computing system 300 includes an arrangement of computer hardware and software components that may be used to implement aspects of the present disclosure. Those skilled in the art will appreciate that the computing system 300 may include more (or fewer) components than those depicted in FIG. 3. It is not necessary, however, that all of these generally conventional components be shown in order to provide an enabling disclosure.

In the illustrated embodiment, the computing system 300 includes a processing unit 302, a network interface 304, a non-transitory computer-readable medium drive 306, and an input/output device interface 308, all of which may communicate with one another by way of a communication bus. The network interface 304 may provide the splice variant determination service 202 (see FIG. 2) with connectivity to one or more networks or computing systems. The processing unit 302 may thus receive information and instructions from other computing devices, systems, or services via a network. The processing unit 302 may also communicate to and from memory 310 and further provide output information via the input/output device interface 308. The input/output device interface 308 may also accept input from various input devices, such as a keyboard, mouse, digital pen, touch screen, etc.

The memory 310 may contain computer program instructions that the processing unit 302 may execute in order to implement one or more embodiments of the present disclosure. The memory 310 generally includes RAM, ROM and/or other persistent or non-transitory computer-readable storage media. The memory 310 may store an operating system 314 that provides computer program instructions for use by the processing unit 302 in the general administration and operation of the splice variant determination service 302. The memory 310 may further include other information for implementing aspects of the present disclosure.

In one embodiment, the memory 310 includes an interface module 312. The interface module 312 can be configured to facilitate generating one or more user interfaces through which data providers 208A, 208B, reference providers 210, or data consumers 212A, 212B utilizing a compatible computing device, may send to, or receive from, the splice variant determination service 202 splice data, reference data, instruction data, metadata, etc., or otherwise communicate with the splice variant determination service 202. Specifically, the interface module 312 can be configured to facilitate processing functions described herein, including obtaining splice data, processing splice data, storing splice data, sending splice data, annotating splice data, etc. For example, data providers 208A, 208B, or data consumers 212A, 212B, may store, annotate, or retrieve junctions determined via a particular assay so that splice variant determination via baseline analysis may be tracked as performed under a consistent assay. This can be done via one or more generated user interfaces. The user interface can be implemented as a graphical user interface (GUI), network-based user interface, computer program, smartphone or table program, or application, touchscreen, wearable computing device interface, command line interface, gesture, voice or text interface, etc., or any combination thereof. Furthermore, the user interfaces can include indicators when a sample has been processed to determine filtered sample junctions that are candidate oncogenic events, or the like.

In addition, the memory 310 may include a data processing module 316 that may be executed by the processing unit 302. In one embodiment, the data processing module 316 implements aspects of the present disclosure. As a non-limiting example, the data processing module 316 can be configured to process splice data, instructions, reference data, or metadata. Specifically, the data processing module 316 can be configured to perform functions described herein, including determining sample junctions, determining baseline junctions, determining filtered sample junctions, determining RNA reads from tissue, removing junction overlap, verifying filtered sample junctions, determining sufficient overlap count, etc.

It should be noted that the splice variant determination service 202 may be implemented by some or all of the components present in the computing system 300 as discussed herein with respect to FIG. 3. In addition, the computing system 300 may include additional components not present in FIG. 3. The modules or components described above may also include additional modules or be implemented by computing devices that may not be depicted in FIG. 2 or 3. For example, although the interface module 312 and the data processing module 316 are identified in FIG. 3 as single modules, one skilled in the relevant art will appreciate that the modules may be implemented by two or more modules and in a distributed manner. Also, although the splice variant determination service 202 and the splice variant determination data store 204 is identified in FIG. 2 as single components one skilled in the relevant art will appreciate that the components may be implemented by two or more components and in a distributed manner. As another example, the computing system 300 and its components may be implemented by network servers, application servers, database servers, combinations of the same, or the like, configured to facilitate data transmission to and from data providers 208A, 208B or data consumers 212A, 212B via, or in lieu of, the network 206. Accordingly, the depictions of the modules and components are illustrative in nature.

**Junctions**

As introduced above, junctions are a way of identifying a particular splice variant. Junctions are identified upstream in the read aligner and are identified by coordinates on the DNA genome. In normal tissues, junctions usually occur at the boundaries between exons (as parts of the DNA sequence that are retained after splicing) rather than introns (parts of the DNA sequence that are spliced out).

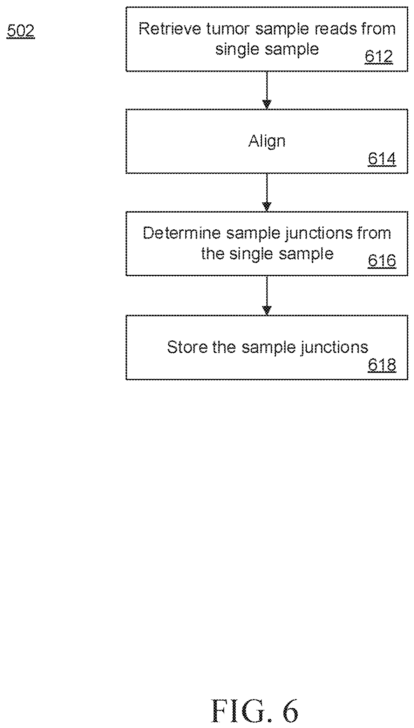

FIG. 4 is a flow diagram illustrating an embodiment of junction analysis implemented by the splice variant determination service 202 (of FIG. 2). Further to FIG. 4, the process of junction analysis 400 begins at block 402, where the splice variant determination service retrieves RNA reads. The RNA reads are nucleotide sequences determined from processing an RNA sample using a sequencer. With reference to FIG. 2, the RNA reads may be retrieved from the splice variant determination data store 204 or from a data provider 208A, 208B. The RNA reads may be determined from a tissue sample and specifically may be from a healthy tissue sample (as discussed further in connection with FIG. 7) or from a tumor tissue sample (as discussed further in connection with FIG. 6). The RNA reads may be determined from a sequencer via the sequencing methods discussed further below.

Further to FIG. 4, at block 404, the RNA reads may be aligned. The RNA reads may be aligned by retrieving RNA reads and aligning the RNA reads to a DNA reference. Alignment determines locations for RNA reads relative to the DNA reference. Referring to FIG. 2, the DNA reference may be provided by the reference providers 210 but stored (and retrieved) locally in the splice variant determination data store 204 for ease of access. Returning to FIG. 4, the reference DNA sequence may be part of a reference genome of a digital nucleic acid sequence database as a representative example of a set of genes for humans and is typically a haploid mosaic of different DNA sequences from multiple donors. The RNA read and the DNA sequence may be aligned using an aligner, such as but not limited to the Bowtie sequence aligner maintained by the Johns Hopkins University of Baltimore, Maryland, USA (described further in connection with Langmead B, Trapnell C, Pop M, Salzberg SL, "Ultrafast and memory-efficient alignment of short DNA sequences to the human genome", Genome Biol 10:R25., incorporated herein by reference in its entirety), the Top Hat sequence aligner maintained by the Johns Hopkins University of Baltimore, Maryland, USA (described further in connection with Trapnell C, Pachter L, Salzberg SL. "TopHat: discovering splice junctions with RNA-Seq", Bioinformatics doi:10.1093/bioinformatics/btp120. , incorporated herein by reference in its entirety) or the STAR sequence aligner maintained on GitHub (described further in connection with Dobin, Davis CA, Schlesinger F, Drenkow J, Zaleski C, Jha S, Batut P, Chaisson M, Gingeras TR., "STAR: ultrafast universal RNA-seq aligner", Bioinformatics. 2013 Jan 1;29(1):15-21. doi: 10.1093/bioinformatics/bts635. Epub 2012 Oct 25, incorporated herein by reference in its entirety). Gaps in the RNA reads aligned to the DNA sequence indicate a splicing event and are used to generate the list of junctions to be processed. In the current implementation, the aligner identifies the splice junctions before downstream processing.

At block 406, the splice variant determination service 202 determines whether there are missing contiguous locations in the RNA read based on a comparison with the aligned DNA sequence. This determination may be performed using an aligner, described above. Also, as introduced above, these missing continuous locations from the RNA read are coordinates on a DNA sequence removed in an aligned RNA sequence. Also, these may occur at the boundaries between exons and introns.

If it is determined that there is a missing contiguous region in the RNA read, then the process of junction analysis 400 proceeds to block 408 where the missing contiguous regions in the RNA read are attributed as a junction.

At block 420, this junction may be stored in the splice variant determination data store 204. This junction may be stored with a notation of the chromosome and the locations in the DNA sequence missing in the RNA read. As a non-limiting example, a junction may be stored as a notation that missing contiguous regions in the RNA read occur at chromosome 21 between positions 12 and 15.

If a missing contiguous region in the RNA read is not detected, then the process of determining junctions proceeds to block 410 and a junction is not attributed to the section under evaluation from the RNA read.

Block 416 encompasses blocks 406, 408, and 410 and may be collectively termed as a process of determining junctions, referenced later at least in connection with FIG. 6 and FIG. 7.

**Oncogenic Junction Determination**

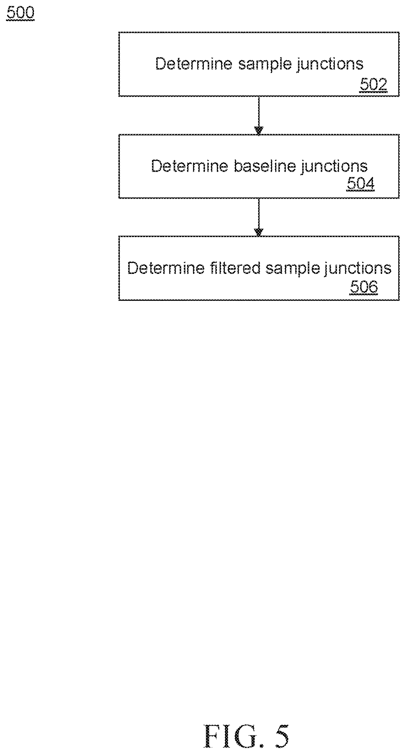

FIG. 5 is a flow diagram illustrating an embodiment of determining possible oncogenic junctions implemented by the splice variant determination service 202 (of FIG. 2). Fig. 5 illustrates an overview of the oncogenic splice variant determination via baseline analysis process discussed in more detail in the previous and following figures.

The process 500 of determining possible oncogenic junctions begins at block 502 where sample junctions are determined. The determination of sample junctions is discussed in further detail in connection with FIG. 6.

Returning to FIG. 5, at block 504, baseline junctions of the baseline reference are determined. The determination of baseline junctions is discuss in further detail in connection with FIG. 7.

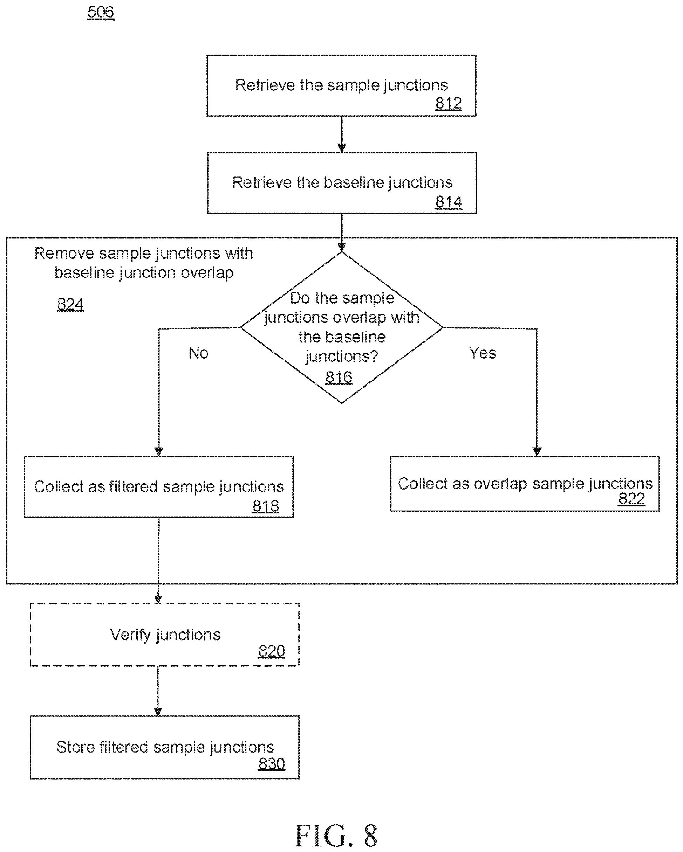

In block 506, filtered sample junctions are determined. The determination of filtered sample junction is discuss in further detail in connection with FIG. 8.

**Sample Junctions**

As introduced above, oncogenic splice variant determination via baseline analysis uses a single tumor sample from a patent and is advantageously simpler than traditional splice variant determinations that use multiple samples (tumor samples and healthy, non-tumor samples) from a patient.

FIG. 6 is a flow diagram illustrating an embodiment of determining sample junctions implemented by the splice variant determination service 202.

The determination of sample junctions 502 illustrated in FIG. 6 begins at block 612 where tumor sample reads reflective of a single tumor sample from a patient is retrieved. The single tumor sample may be collected from tumor tissue for identification of abnormal junctions indicative of an abnormal splice variant. In certain embodiments, the tumor sample reads may be determined in a conventional manner from sequencing the single tumor sample, as discussed further below in connection with sequencing methods. In certain embodiments, the tumor sample reads may be retrieved from the data providers 208A, 208B, (discuss further in connection with FIG. 2) where the data providers either produce the tumor sample reads themselves (such as, but not limited to, via sequencing methods discussed further below) or are a repository for the tumor sample reads from where the splice variant determination service retrieves the tumor sample reads.

Further to FIG. 6, at block 614, the tumor sample RNA reads are aligned to a DNA reference. The tumor sample RNA reads may be determined via an aligner, discussed further above in connection with FIG. 4.

Further to FIG. 6, at block 616, sample junctions are determined from the tumor sample RNA reads of block 614. The sample junctions may be determined via an aligner, discussed further above in connection with FIG. 4. As an illustrative and non-limiting example with reference to FIG. 4, the sample junctions may be determined similar to the determining junctions block 416 of the process of junctions analysis 400 as illustrated in connection with FIG. 4, where the retrieved RNA reads in block 402 are the RNA reads determined from the single sample in block 614 and the junctions attributed in block 408 are the sample junctions determined from block 616.

Further to FIG. 6, at block 618, the sample junctions may be stored in the splice variant determination data store 204, discussed further in connection with FIG. 2, for further retrieval and processing by the splice variant determination service 202.

In certain embodiments, determination of sample junctions may occur live with other processes (such as, but not limited to the determination of filtered sample junctions and/or the determination of baseline junctions) performed by the splice variant determination service 202 during a session of oncogenic splice variant determination via baseline analysis. In other embodiments, the determination of sample junctions may be performed independently, later, or earlier than other processes (such as, but not limited to the determination of filtered sample junctions and/or the determination of baseline junctions) performed by the splice variant determination service 202 during a session of oncogenic splice variant determination via baseline analysis.

**Baseline Junctions**

As introduced above, oncogenic splice variant determination via baseline analysis is largely directed to junction calling for oncogenic events, not de-novo splice variant calling. A number of errors may be introduced when splice variant determination is performed via de-novo splice variant calling. These errors may include algorithm or assay issues that may hinder the accuracy of splice variant calling for oncogenic events. As a non-limiting example, since the tumor samples being considered are FFPE, there may be artifacts introduced by the assay or sample preparation in de-novo splice variant calling. Also, since de-novo splice variant calling relies on read alignments using RNA aligners, there may be alignment artifacts.

However, inherently, there is a problem that the transcriptome has not been comprehensively annotated, leading to events reported which are irrelevant to tumor progression. These may be real constitutive events in normal, healthy cells that have not been characterized. Algorithm or assay issues could hypothetically be handled by eliminating errors from the assay and algorithms. However, it may be difficult to remove errors due to not comprehensively annotating the transcriptome without attempting to characterize what is actually in normal, healthy tissue.

Furthermore, as noted above, de-novo splice variant calling typically requires at least two samples from a single patient (at least one sample from healthy tissue and at least one sample from tumor tissue). Having to process additional samples is invasive and clinically undesirable. Also, running multiple samples for a single patient drastically increases both the reagent and sequencing costs.

Accordingly, at least these drawbacks of traditional de-novo splice variant calling may be overcome when performing oncogenic splice variant determination via baseline analysis. Baseline analysis refers to an analysis using a baseline reference of a diverse cross section of baseline junctions from normal, healthy, non-tumor tissue samples used as a reference when evaluating a single sample from a patient. These cross sections can be across any number of criteria, such as but not limited to geographical region, age, gender, ethic group, FFPE artifacts, FFPE quality, homolog artifacts, polymerase read-through artifacts, non-oncological alternative splicing, tissue type or the like. A cross section may be a variation within a particular criteria. For example, a cross section of age may include samples from a diversity of donors across different ages, including (but not limited to) ages 1, 5, 10, 15, 20, 25, 30, 35, 40, 45, 50, 55, 60, 65, 70, 75, 80, 85, 90, 95, 100, 105, 110, and the like. As a further example, a cross section of tissue type may include tissue from different body parts, including (but not limited to) tissue from various locations from a lung, adrenal gland, bladder, breast, ovary, liver, prostate, skin, spleen, and the like. As a further example, a cross section of FFPE artifacts may include (but is not limited to) different values of deamination, fragmentation, base modification, abasic sites, and the like. As a further example, a cross section of FFPE quality may include (but is not limited to) different samples with fragments of RNA of different sizes.

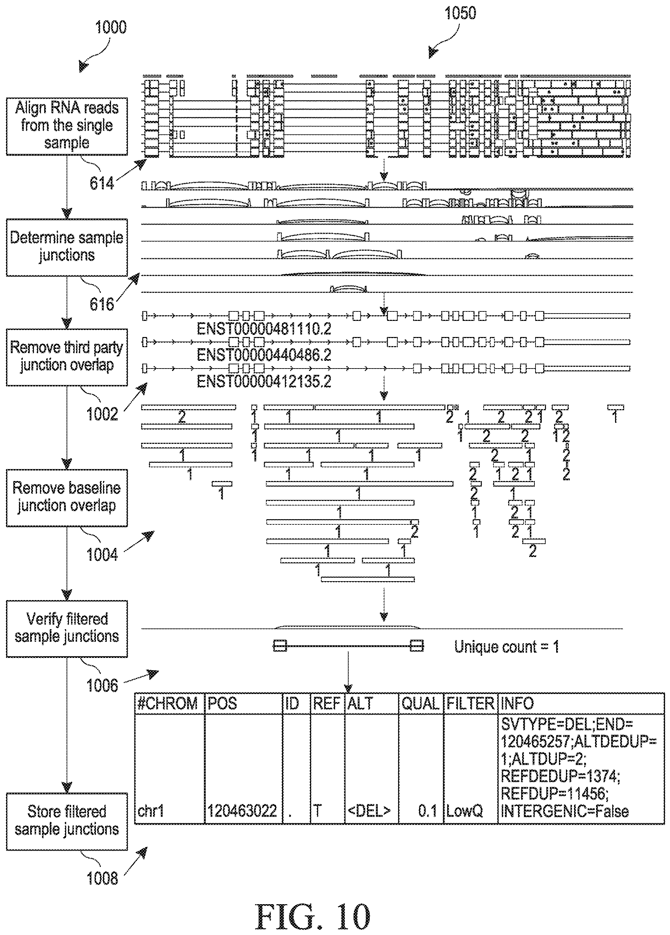

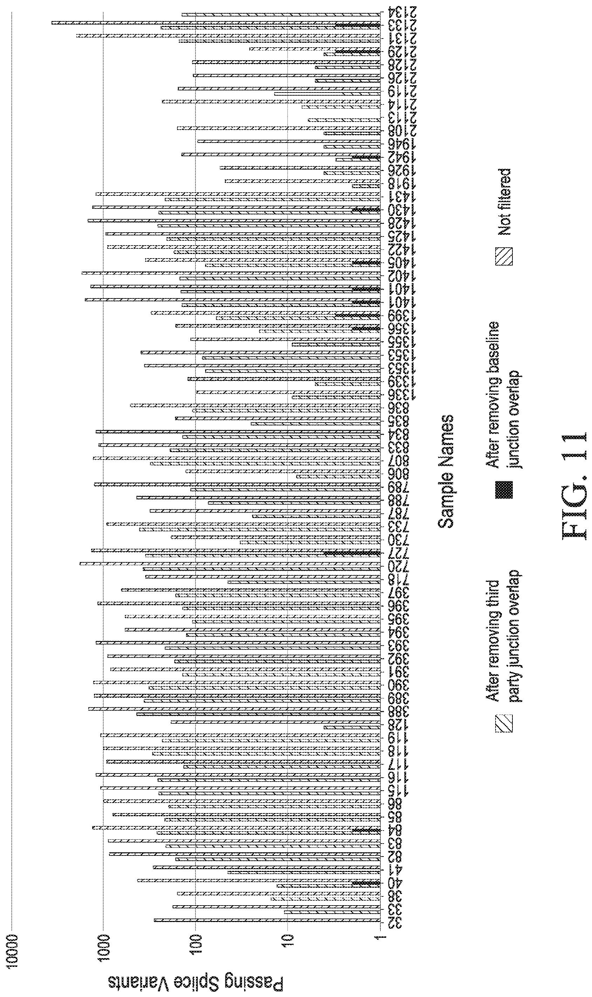

Furthermore, the baseline reference may to be comprehensive enough to capture the constitutive splicing for many different tissue types. Even though samples in the baseline reference may be from many different tissue types which may not have completely overlapping splice variant expression, there would be significant and sufficient overlap in the types of splice variants found across tissues for the baseline analysis to be effective as noted in connection with FIG. 10 and FIG. 11. It can be more effective to reduce spurious or normal physiological splice junctions to be mis-identified as oncogenic events with a more comprehensive baseline reference.

Splice variant determination via baseline analysis may come at a cost to sensitivity since real oncogenic splicing events which overlap with assay or alignment errors will also be filtered. However, as alignment and sample handling improves, the baseline reference can also be updated to reflect improved methods while capturing normal constitutive junctions from normal, healthy (non-tumor) samples.

FIG. 7 is a flow diagram illustrating an embodiment of determining baseline junctions of a baseline reference implemented by the splice variant determination service 202.

The determination of baseline junctions 504 illustrated in FIG. 7 begins at block 712 where healthy sample reads from a cross section of healthy (non-tumor tissue) samples are retrieved. As noted above, the cross section may be any cross section of junctions from healthy (non-tumor tissue) samples used as a reference when evaluating a single sample from a patient. These cross sections can be across any number of criteria, such as but not limited to geographical region, age, gender, ethic group, FFPE artifacts, FFPE quality, homolog artifacts, polymerase read-through artifacts, non-oncological alternative splicing, tissue type, or the like.

In certain embodiments, the healthy sample reads may be determined in a conventional manner from sequencing the individual healthy tissue samples. Sequencing methods are discussed further below. In certain embodiments, the healthy sample reads may be retrieved from the data providers 208A, 208B, (discuss further in connection with FIG. 2) where the data providers either produce the healthy sample data themselves (such as, but not limited to, via sequencing discussed further below) or are a repository for the healthy sample reads from where the splice variant determination service retrieves the healthy sample reads.

Further to FIG. 7, at block 714, healthy sample reads are aligned with a reference sequence. The healthy sample reads may be aligned via an aligner, discussed further above in connection with FIG. 4.

At block 716, baseline junctions are determined from the healthy sample RNA reads of block 714. The baseline junctions may be determined via an aligner, discussed further above in connection with FIG. 4. As an illustrative and non-limiting example with reference to FIG. 4, the baseline junctions may be determined similar to the determining junctions block 416 of junction analysis 400 as illustrated in connection with FIG. 4, where the retrieved RNA reads in block 402 are the healthy sample reads determined from the healthy sample in block 712 and the junctions attributed in block 408 are the baseline junctions determined from block 716.

Further to FIG. 7, at block 718, the collection of baseline junctions may be stored as a baseline reference in the splice variant determination data store 204, discussed further in connection with FIG. 2, for further retrieval and processing by the splice variant determination service 202.

In certain embodiments, determination of baseline junctions or the baseline reference may be performed prior to the determination of filtered sample junctions and/or the determination of sample junctions. Accordingly, savings in computing resources may be realized when the baseline reference is retrieved as needed from the splice variant determination data store 204 rather than being determined on the fly or ad hoc with each session of splice variant determination via baseline analysis.

Furthermore, in particular embodiments, determination of baseline junctions may include retrieval of a stored baseline reference from the splice variant determination data store 204. In further embodiments, determination of the baseline reference and constituting baseline junctions may occur live during a session of oncogenic splice variant determination via baseline analysis.

**Filtered Sample Junctions**

Splice variant determination via baseline analysis produces filtered sample junctions. These filtered sample junctions may indicate sample junctions as possible oncogenic splice variants. Filtered sample junctions may be sample junctions that do not overlap with the baseline junctions, when the sample junctions and the baseline junctions are determined using a same assay. Also, due to being junctions not known to result from healthy, non-oncogenic, tissue, the filtered sample junctions may be identified as novel and thus possibly oncogenic or likely to be associated with cancer. These filtered sample junctions may be identified as splice variants and potential targets for drug therapies.

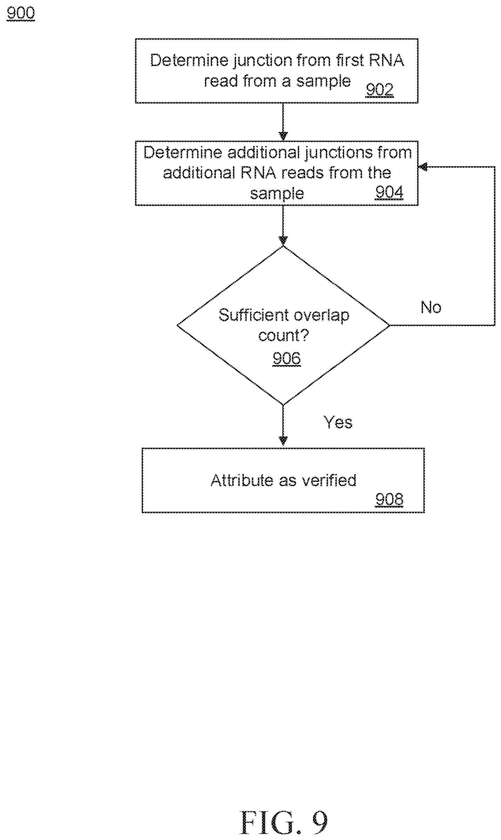

In certain embodiments, filtered sample junctions may be additionally verified by evidence that supports how a filtered sample junction is not erroneous. This type of filtered sample junction may be a verified filtered sample junction, discussed further in connection with FIG. 9.

In additional embodiments, filtered sample junctions may be determined as sample junctions that do not overlap with third party junctions, in addition to not overlapping with baseline junctions as discussed above. This type of filtered sample junction may be termed as a baseline third party filtered sample junction, discussed further in connection with FIG. 10. Also, as discussed further in connection with FIG. 10, this type of filtered sample junction may be additionally verified and may be termed as a verified baseline third party filtered sample junction.

FIG. 8 is a flow diagram illustrating an embodiment of determining filtered sample junctions implemented by the splice variant determination service 202. The determination of filtered sample junctions 506 illustrated in FIG. 7 begins at block 812 where sample junctions are retrieved. The determination of the sample junctions is discussed further in connection with FIG. 6. Also, the sample junctions may be retrieved from the splice variant determination data store 204, as discussed further in connection with FIG. 2 and FIG. 6.

Returning to FIG. 8, at block 814, the baseline reference of baseline junctions (determined using a common assay with the sample junctions of block 812) are retrieved. The determination of the baseline reference is discussed further in connection with FIG. 7. Also, the baseline reference may be retrieved from the splice variant determination data store 204, as discussed further in connection with FIG. 2 and FIG. 7.

At block 816, the splice variant determination service 202 determines whether the sample junctions overlap with the baseline junctions. In certain embodiments, this determination may be based on comparing the values of each sample junction with each of the baseline junctions of the baseline reference to determine whether they overlap. Overlap refers to determining that there are same values, or coordinates between the junctions being compared. As a non-liming example, this may be done where a first sample junction is compared to each of the baseline junctions before a second sample junction is compared to each of the baseline junctions.

Further to block 816, in certain embodiments, all baseline junctions of a baseline reference may be individually referenced to determine whether there is overlap with sample junctions in block 816. However, in further embodiments, the baseline junctions referenced may be dependent upon the coordinates of the sample junctions retrieved in block 812. Specifically, baseline junctions that could overlap with the sample junctions retrieved in block 812 are referenced while baseline junctions that would not overlap with the sample junctions determined in block 812 are not referenced. As a non-limiting example, sample junctions of a particular chromosome may be compared with baseline junctions of that chromosome. Advantageously, having referenced baseline junctions dependent upon the coordinates of sample junctions may improve computational efficiency when compared with retrieving or processing all baseline junctions of a baseline reference independent of the coordinates of the sample junctions.

If it is determined that a sample junction overlaps with a baseline junction of the baseline reference, then the process proceeds to block 822 where the overlapping sample junction is collected as an overlap sample junction and not a filtered sample junction.

If it is determined that a sample junction does not overlap with any of the baseline junctions of the baseline reference, then the process proceeds to block 818 where the sample junction that does not overlap with any of the baseline junctions is collected as a filtered sample junction.

Block 824 refers to a collective step of collecting filtered sample junctions by removing sample junctions with baseline junction overlap, and is a restatement of block 816, block 818, and block 822 collectively. Block 822 may be referred to later in connection with FIG. 10.

At block 820, filtered sample junctions are verified. The verification of filtered sample junctions is discussed further in connection with FIG. 9. In certain embodiments, the verification of filtered sample junctions may be optional (as noted by the dotted lines of block 820) and filtered sample junctions may be used without verification as described in connection with FIG. 9. Alternatively, verification may occur at other parts of the process of splice variant determination via baseline analysis such as, but not limited to any point after which junctions are determined.

Further to FIG. 8, at block 830, the filtered sample junctions may be stored in the splice variant determination data store 204, discussed further in connection with FIG. 2, for further retrieval and processing by the splice variant determination service 202 or for further retrieval and processing by the data consumers 208A, 208B.

## Verification

Junction verification may be performed in order to determine whether a filtered sample junction that does not overlap with any baseline junction is erroneous. In many embodiments, junction verification may be performed on filtered sample junctions determined via block 824 of FIG. 8, where sample junctions that do not overlap with baseline junctions are collected as filtered sample junctions. Doing so may be advantageous as, if sample junctions are to be verified, the number of filtered sample junctions may be smaller than the number of sample junctions. However, further embodiments also contemplate verification of sample junctions and not filtered sample junctions and/or verification of both sample junctions and filtered sample junctions.

As noted above, junctions may be determined via at least one RNA read from a single sample. As explained further below in connection with sequencing methods, RNA from a sample may be amplified, or duplicated, during the course of sequencing. The amplified RNA may be utilized to increase a signal to noise ratio during sequencing. In addition, reads from the amplified RNA may be utilized to confirm, or support, a particular read from the RNA. Similarly, the reads from the amplified RNA may be a supporting junction read that confirms, or supports, the accuracy of a particular junction determined from the RNA read. These supporting junction reads may be reads that include junctions from additional reads that are redundant with a particular junction determined from the RNA read. Accordingly, verification of a particular junction may be determined when a threshold number of supporting junction reads are determined for a particular junction under verification.

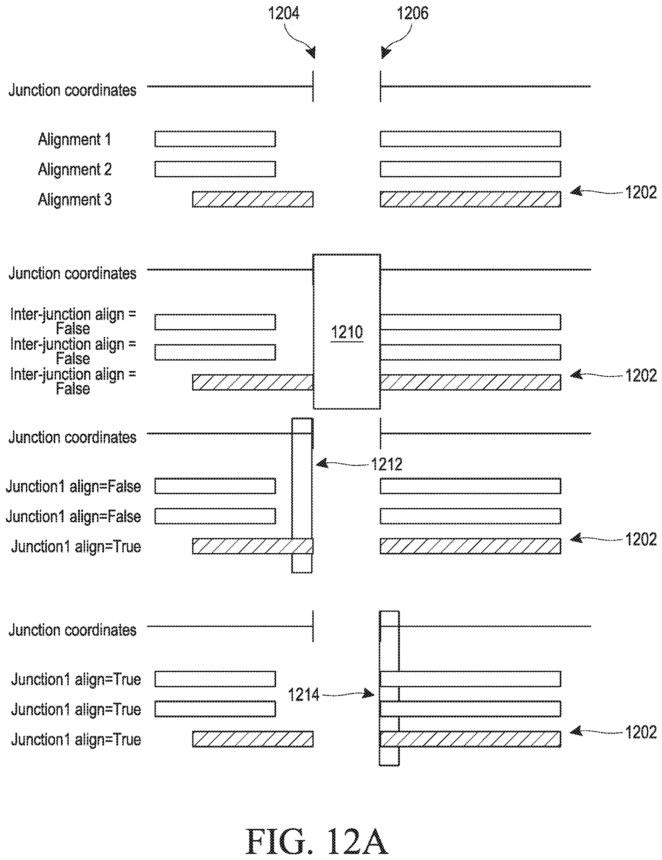

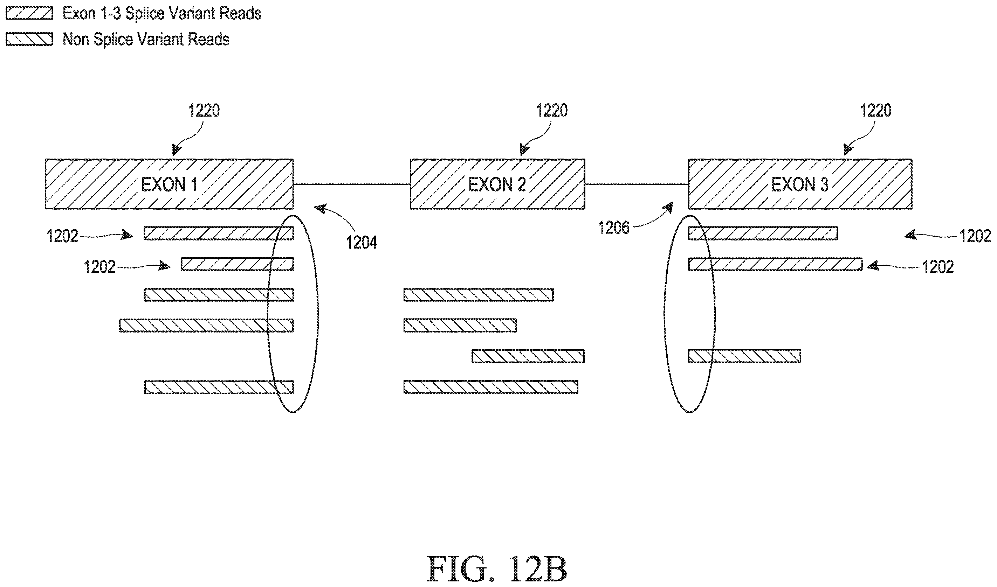

FIG. 12A and FIG. 12B are conceptual diagrams illustrating features of verifying filtered sample junctions. As illustrated in FIG. 12A and FIG. 12B, a supporting junction read 1202 may be a split read where alignment ends at a start 1204 of a junction under verification and starts again at the other end 1206 of the junction under verification. This may be determined by evaluating 1210 whether alignment spans the junction under verification, evaluating 1212 whether alignment ends at one end of the junction under verification, and/or evaluating 1214 whether alignment starts at the other end of the junction under verification.

Accordingly, as illustrated in FIG. 12A, a read would not be not counted as a supporting junction read if there is any aligned area of the read within the junction. Also, as stated another way and illustrated in FIG. 12B, an exon 1220 must align to the ends of the junction under verification not align in the middle of the junction under verification.

In certain embodiments, junctions may be verified by attributing a score to a junction under verification. The score may be from 0-1 where .1 point is added for each supporting junction read, as expressed with the equation:

$$\text{score} = (\min(u,M) - N) * 1/(M\text{-}N),$$

where M = maximum number of reads that span a junction under verification (default 10), N = minimum number of reads that span a junction under verification (default 0), u = number of supporting junction reads. As espoused by this equation, verification is achieved when at least 10 supporting junction reads are determined for a junction under verification.

FIG. 9 is a flow diagram illustrating an embodiment of verifying junctions implemented by the splice variant determination service 202. The process of verifying junctions 900 illustrated in FIG. 9 begins at block 902 where a junction from a first RNA read from a sample is determined. In particular embodiments, the sample may be the single sample discussed further above in connection with FIG. 6 and the junctions determined from the single sample as discussed in connection with blocks 612, 614, and 616. Also, a junction under verification analysis may be the junction determined from the first RNA read. Also, the process of determining junctions is discussed further in connection with FIG. 4.

Further to FIG. 9, at block 904, additional junctions from RNA reads may be determined from the sample. As discussed above, a single sample may have multiple RNA reads. These RNA reads may be utilized as supporting junction reads that include junctions redundant with the first read. Also, the sample may be the single sample discussed further above in connection with FIG. 6 and the junctions determined from the single sample as discussed in connection with blocks 612, 614, and 616. Furthermore, the process of determining junctions is discussed further in connection with FIG. 4.

Further to FIG. 9, at block 906, the splice variant determination service 202 determines whether a sufficient overlap count is present from the additional junctions from the additional RNA reads. The sufficient overlap count may be a threshold count of overlapping supporting junction reads from which verification may be attributed (such as, but not limited to 2, 3, 4, 5, 6, 7, 8, 9, or 10 overlapping supporting junction reads).

If it is determined that sufficient overlap count is present, then the process proceeds to block 908 where the junction referenced in block 902 is attributed as verified (or a verified filtered sample junction).

If it is determined that the sufficient overlap count not present, then the process returns to block 904 where additional junctions from RNA reads may be determined from the sample.

**Exemplary Embodiment**

FIG. 10 is a flow diagram with accompanying conceptual illustration of an embodiment of determining possible oncogenic junctions. The flow diagram of FIG. 10 illustrates an embodiment in which third party junctions that are indicative of non-cancerous splice variants are utilized as part of splice variant determination via baseline analysis. These third party junctions that are indicative of non-cancerous splice variants may be determined from de-novo splice variant calling, in contrast with baseline junctions that are determined from a cross sample of health (non-tumor tissue) samples.

Juxtaposed to the flow diagram 1000 are illustrations 1050 that represent each of the blocks of flow diagram 1000.

The process of oncogenic splice variant determination 1000 illustrated in FIG. 10 begins at block 614 where RNA reads from the single tumor sample are aligned, as discussed above in connection with FIG. 6.

At block 616, sample junctions are determined from the RNA reads of block 614, as discussed further above in connection with FIG. 6.

At block 1002, sample junctions that overlap with third party junctions are removed. As discussed above, these third party junctions that are indicative of non-cancerous splice variants may be determined from de-novo splice variant calling, in contrast with the baseline reference of baseline junctions that are determined from a cross sample of healthy (non-tumor tissue) samples. Removal of sample junctions that overlap with third party junctions in accordance with block 1002 may be performed in a manner similar to the process of removing sample junction and baseline junction overlap 824 as discussed in connection with FIG. 8, but where the baseline junctions (of FIG. 8) are the third party junctions and the filtered sample junctions (of FIG. 8) are the third party filtered sample junctions remaining after removal of the sample junctions that overlap with third party junctions.

Further to FIG. 10, at block 1004, baseline third party filtered sample junctions are collected by removing third party filtered sample junctions with baseline junction overlap. Removal of third party filtered sample junctions that overlap with baseline junctions in accordance with block 1004 may be performed in a manner similar to the process of removing sample junction and baseline junction overlap 824 as discussed in connection with FIG. 8, but where the sample junctions (of FIG. 8) are the third party filtered sample junctions and the filtered sample junctions (of FIG. 8) are the baseline third party filtered sample junctions remaining after removal of the third party filtered sample junctions that overlap with baseline junctions.

Further to FIG. 10, at block 1006, the baseline third party filtered sample junctions are verified. Verification of baseline third party filtered sample junctions in accordance with block 1006 may be performed in a manner similar to the process of verifying junctions 900 as discussed in connection with FIG. 9, but where the junction from the first RNA read (of FIG. 9) is a baseline third party filtered sample junction and the junction attributed as verified in block 908 is a verified baseline third party filtered sample junction.

At block 1008, the verified baseline third party filtered sample junctions may be stored. Storage of the verified baseline third party filtered sample junction may be performed in a manner similar to the storage of filtered sample junctions discussed in connection with block 830 of FIG. 8, but where the verified baseline third party filtered sample junction are stored, rather than the filtered sample junctions. The verified baseline third party filtered sample junctions may be stored in any data structure such as, but not limited to, a Variant Call Format (VCF) file in the illustrated embodiment. A VCF file contains at least meta-information lines, a header line, and then data lines each containing coordinates associated with at least one verified filtered sample junction.

As noted above, filtered sample junctions (such as the verified baseline third party filtered sample junctions as discussed above) may be determined as sample junctions that do not overlap with third party junctions, in addition to not overlapping with baseline junctions as discussed above. Although block 1002, block 1004, and block 1006 occur in a particular order within the flow diagram 1000 of FIG. 10, block 1002, block 1004, and block 1006 may occur at any point of determining filtered sample junctions with dependencies adjusted accordingly.

FIG. 11 is a table illustrating experimental results from the embodiment of FIG. 10. As illustrated in FIG. 11, splice variants (junctions) are plotted against 71 different cross validated normal, healthy (non-tumor) samples across a cross section of tissue type (lung, adrenal gland, bladder, breast, ovary, liver, prostate, skin, and spleen). Seven different cross validation sets were generated consisting of 10 samples to test and generating a baseline reference from the remaining 61. Filtering is performed by first removing third party junction overlap and then removing baseline junction overlap. As noted in FIG. 11, there is a lower number of splice variants after removing baseline junction overlap relative to after removing third party junction overlap. This indicates a greater decrease in the number of novel junctions (filtered sample junctions, or candidate oncogenic events) as compared with removal of sample junctions that overlap with third party junctions. Indeed, very few novel junctions remain as filtered sample junctions after undergoing oncogenic splice variant determination via baseline analysis.

## Performance/Limit of Detection

The limit of detection for variants in RNA may be a function of how much of the affected transcript is expressed in addition to the specific splice variant expressed. The effective limit of detection in fusion copy number per ng of RNA may be detected using digital droplet PCR (ddPCR) to estimate how much of a splice variant transcript is expressed in a particular FFPE sample.

To demonstrate the performance of oncogenic splice variant determination via baseline analysis, three splice variants (EGFRviii, ARv7, and MET exon 14 skipping) were identified in FFPE tumor samples and then measured using ddPCR. If the expression level of the splice variant was high enough, these samples were then titrated down to 2 copies per ng of RNA. From this data, oncogenic splice variant determination via baseline analysis called at least one of the splice variants with as low as .13 copies per ng of RNA (EGFRviii). At 5 copies per ng of RNA, all three splice variants are correctly identified via oncogenic splice variant determination via baseline analysis.

## Sequencing Methods

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleotide base type from another are particularly applicable. In some embodiments, the process to determine the nucleotide sequence of a target nucleic acid can be an automated process. Preferred embodiments include sequencing-by-synthesis ("SBS") techniques.

SBS techniques generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

SBS can utilize nucleotide monomers that have a terminator moiety or those that lack any terminator moieties. Methods utilizing nucleotide monomers lacking terminators include, for example, pyrosequencing and sequencing using y-phosphate-labeled nucleotides, as set forth in further detail below. In methods using nucleotide monomers lacking terminators, the number of nucleotides added in each cycle is generally variable and dependent upon the template sequence and the mode of nucleotide delivery. For SBS techniques that utilize nucleotide monomers having a terminator moiety, the terminator can be effectively irreversible under the sequencing conditions used as is the case for traditional Sanger sequencing which utilizes dideoxynucleotides, or the terminator can be reversible as is the case for sequencing methods developed by Solexa (now Illumina, Inc.).

SBS techniques can utilize nucleotide monomers that have a label moiety or those that lack a label moiety. Accordingly, incorporation events can be detected based on a characteristic of the label, such as fluorescence of the label; a characteristic of the nucleotide monomer such as molecular weight or charge; a byproduct of incorporation of the nucleotide, such as release of pyrophosphate; or the like. In embodiments, where two or more different nucleotides are present in a sequencing reagent, the different nucleotides can be distinguishable from each other, or alternatively, the two or more different labels can be the indistinguishable under the detection techniques being used. For example, the different nucleotides present in a sequencing reagent can have different labels and they can be distinguished using appropriate optics as exemplified by the sequencing methods developed by Solexa (now Illumina, Inc.).

Preferred embodiments include pyrosequencing techniques. Pyrosequencing detects the release of inorganic pyrophosphate (PPi) as particular nucleotides are incorporated into the nascent strand (Ronaghi, M., Karamohamed, S., Pettersson, B., Uhlen, M. and Nyren, P. (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 11(1), 3-11; Ronaghi, M., Uhlen, M. and Nyren, P. (1998) "A sequencing method based on real-time pyrophosphate." Science 281(5375), 363; U.S. Pat. No. 6,210,891; U.S. Pat. No. 6,258,568 and U.S. Pat. No. 6,274,320, the disclosures of which are incorporated herein by reference in their entireties). In pyrosequencing, released PPi can be detected by being immediately converted to adenosine triphosphate (ATP) by ATP sulfurylase, and the level of ATP generated is detected via luciferase-produced photons. The nucleic acids to be sequenced can be attached to features in an array and the array can be imaged to capture the chemiluminscent signals that are produced due to incorporation of a nucleotides at the features of the array. An image can be obtained after the array is treated with a particular nucleotide type (e.g. A, T, C or G). Images obtained after addition of each nucleotide type will differ with regard to which features in the array are detected. These differences in the image reflect the different sequence content of the features on the array. However, the relative locations of each feature will remain unchanged in the images. The images can be stored, processed and analyzed using the methods set forth herein. For example, images obtained after treatment of the array with each different nucleotide type can be handled in the same way as exemplified herein for images obtained from different detection channels for reversible terminator-based sequencing methods.

In another exemplary type of SBS, cycle sequencing is accomplished by stepwise addition of reversible terminator nucleotides containing, for example, a cleavable or photobleachable dye label as described, for example, in WO 04/018497 and U.S. Pat. No. 7,057,026, the disclosures of which are incorporated herein by reference. This approach is being commercialized by Solexa (now Illumina Inc.), and is also described in WO 91/06678 and WO 07/123,744, each of which is incorporated herein by reference. The availability of fluorescently-labeled terminators in which both the termination can be reversed and the fluorescent label cleaved facilitates efficient cyclic reversible termination (CRT) sequencing. Polymerases can also be co-engineered to efficiently incorporate and extend from these modified nucleotides.

Preferably in reversible terminator-based sequencing embodiments, the labels do not substantially inhibit extension under SBS reaction conditions. However, the detection labels can be removable, for example, by cleavage or degradation. Images can be captured following incorporation of labels into arrayed nucleic acid features. In particular embodiments, each cycle involves simultaneous delivery of four different nucleotide types to the array and each nucleotide type has a spectrally distinct label. Four images can then be obtained, each using a detection channel that is selective for one of the four different labels. Alternatively, different nucleotide types can be added sequentially and an image of the array can be obtained between each addition step. In such embodiments each image will show nucleic acid features that have incorporated nucleotides of a particular type. Different features will be present or absent in the different images due the different sequence content of each feature. However, the relative position of the features will remain unchanged in the images. Images obtained from such reversible terminator-SBS methods can be stored, processed and analyzed as set forth herein. Following the image capture step, labels can be removed and reversible terminator moieties can be removed for subsequent cycles of nucleotide addition and detection. Removal of the labels after they have been detected in a particular cycle and prior to a subsequent cycle can provide the advantage of reducing background signal and crosstalk between cycles. Examples of useful labels and removal methods are set forth below.

In particular embodiments some or all of the nucleotide monomers can include reversible terminators. In such embodiments, reversible terminators/cleavable fluors can include fluor linked to the ribose moiety via a 3' ester linkage (Metzker, Genome Res. 15:1767-1776 (2005), which is incorporated herein by reference). Other approaches have separated the terminator chemistry from the cleavage of the fluorescence label (Ruparel et al., Proc Natl Acad Sci USA 102: 5932-7 (2005), which is incorporated herein by reference in its entirety). Ruparel et al described the development of reversible terminators that used a small 3' allyl group to block extension, but could easily be deblocked by a short treatment with a palladium catalyst. The fluorophore was attached to the base via a photocleavable linker that could easily be cleaved by a 30 second exposure to long wavelength UV light. Thus, either disulfide reduction or photocleavage can be used as a cleavable linker. Another approach to reversible termination is the use of natural termination that ensues after placement of a bulky dye on a dNTP. The presence of a charged bulky dye on the dNTP can act as an effective terminator through steric and/or electrostatic hindrance. The presence of one incorporation event prevents further incorporations unless the dye is removed. Cleavage of the dye removes the fluor and effectively reverses the termination. Examples of modified nucleotides are also described in U.S. Pat. No. 7,427,673, and U.S. Pat. No. 7,057,026, the disclosures of which are incorporated herein by reference in their entireties.

Additional exemplary SBS systems and methods which can be utilized with the methods and systems described herein are described in U.S. Patent Application Publication No. 2007/0166705, U.S. Patent Application Publication No. 2006/0188901, U.S. Pat. No. 7,057,026, U.S. Patent Application Publication No. 2006/0240439, U.S. Patent Application Publication No. 2006/0281109, PCT Publication No. WO 05/065814, U.S. Patent Application Publication No.

2005/0100900, PCT Publication No. WO 06/064199, PCT Publication No. WO 07/010,251, U.S. Patent Application Publication No. 2012/0270305 and U.S. Patent Application Publication No. 2013/0260372, the disclosures of which are incorporated herein by reference in their entireties.

Some embodiments can utilize detection of four different nucleotides using fewer than four different labels. For example, SBS can be performed utilizing methods and systems described in the incorporated materials of U.S. Patent Application Publication No. 2013/0079232. As a first example, a pair of nucleotide types can be detected at the same wavelength, but distinguished based on a difference in intensity for one member of the pair compared to the other, or based on a change to one member of the pair (e.g. via chemical modification, photochemical modification or physical modification) that causes apparent signal to appear or disappear compared to the signal detected for the other member of the pair. As a second example, three of four different nucleotide types can be detected under particular conditions while a fourth nucleotide type lacks a label that is detectable under those conditions, or is minimally detected under those conditions (e.g., minimal detection due to background fluorescence, etc.). Incorporation of the first three nucleotide types into a nucleic acid can be determined based on presence of their respective signals and incorporation of the fourth nucleotide type into the nucleic acid can be determined based on absence or minimal detection of any signal. As a third example, one nucleotide type can include label(s) that are detected in two different channels, whereas other nucleotide types are detected in no more than one of the channels. The aforementioned three exemplary configurations are not considered mutually exclusive and can be used in various combinations. An exemplary embodiment that combines all three examples, is a fluorescent-based SBS method that uses a first nucleotide type that is detected in a first channel (e.g. dATP having a label that is detected in the first channel when excited by a first excitation wavelength), a second nucleotide type that is detected in a second channel (e.g. dCTP having a label that is detected in the second channel when excited by a second excitation wavelength), a third nucleotide type that is detected in both the first and the second channel (e.g. dTTP having at least one label that is detected in both channels when excited by the first and/or second excitation wavelength) and a fourth nucleotide type that lacks a label that is not, or minimally, detected in either channel (e.g. dGTP having no label).

Further, as described in the incorporated materials of U.S. Patent Application Publication No. 2013/0079232, sequencing data can be obtained using a single channel. In such so-called one-dye sequencing approaches, the first nucleotide type is labeled but the label is removed after the first image is generated, and the second nucleotide type is labeled only after a first image is generated. The third nucleotide type retains its label in both the first and second images, and the fourth nucleotide type remains unlabeled in both images.

Some embodiments can utilize sequencing by ligation techniques. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. As with other SBS methods, images can be obtained following treatment of an array of nucleic acid features with the labeled sequencing reagents. Each image will show nucleic acid features that have incorporated labels of a particular type. Different features will be present or absent in the different images due the different sequence content of each feature, but the relative position of the features will remain unchanged in the images. Images obtained from ligation-based sequencing methods can be stored, processed and analyzed as set forth herein. Exemplary SBS systems and methods which can be utilized with the methods and systems described herein are described in U.S. Pat. No. 6,969,488, U.S. Pat. No. 6,172,218, and U.S. Pat. No. 6,306,597, the disclosures of which are incorporated herein by reference in their entireties.

Some embodiments can utilize nanopore sequencing (Deamer, D. W. & Akeson, M. "Nanopores and nucleic acids: prospects for ultrarapid sequencing." Trends Biotechnol. 18, 147-151 (2000); Deamer, D. and D. Branton, "Characterization of nucleic acids by nanopore analysis". Acc. Chem. Res. 35:817-825 (2002); Li, J., M. Gershow, D. Stein, E. Brandin, and J. A. Golovchenko, "DNA molecules and configurations in a solid-state nanopore microscope" Nat. Mater. 2:611-615 (2003), the disclosures of which are incorporated herein by reference in their entireties). In such embodiments, the target nucleic acid passes through a nanopore. The nanopore can be a synthetic pore or biological membrane protein, such as $\alpha$-hemolysin. As the target nucleic acid passes through the nanopore, each base-pair can be identified by measuring fluctuations in the electrical conductance of the pore. (U.S. Pat. No. 7,001,792; Soni, G. V. & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2, 459-481 (2007); Cockroft, S. L., Chu, J., Amorin, M. & Ghadiri, M. R. "A single-molecule nanopore device detects DNA polymerase activity with single-nucleotide resolution." J. Am. Chem. Soc. 130, 818-820 (2008), the disclosures of which are incorporated herein by reference in their entireties). Data obtained from nanopore sequencing can be stored, processed and analyzed as set forth herein. In particular, the data can be treated as an image in accordance with the exemplary treatment of optical images and other images that is set forth herein.

Some embodiments can utilize methods involving the real-time monitoring of DNA polymerase activity. Nucleotide incorporations can be detected through fluorescence resonance energy transfer (FRET) interactions between a fluorophore-bearing polymerase and $\gamma$-phosphate-labeled nucleotides as described, for example, in U.S. Pat. No.

7,329,492 and U.S. Pat. No. 7,211,414 (each of which is incorporated herein by reference) or nucleotide incorporations can be detected with zero-mode waveguides as described, for example, in U.S. Pat. No. 7,315,019 (which is incorporated herein by reference) and using fluorescent nucleotide analogs and engineered polymerases as described, for example, in U.S. Pat. No. 7,405,281 and U.S. Patent Application Publication No. 2008/0108082 (each of which is incorporated herein by reference). The illumination can be restricted to a zeptoliter-scale volume around a surface-tethered polymerase such that incorporation of fluorescently labeled nucleotides can be observed with low background (Levene, M. J. et al. "Zero-mode waveguides for single-molecule analysis at high concentrations." Science 299, 682-686 (2003); Lundquist, P. M. et al. "Parallel confocal detection of single molecules in real time." Opt. Lett. 33, 1026-1028 (2008); Korlach, J. et al. "Selective aluminum passivation for targeted immobilization of single DNA polymerase molecules in zero-mode waveguide nano structures." Proc. Natl. Acad. Sci. USA 105, 1176-1181 (2008), the disclosures of which are incorporated herein by reference in their entireties). Images obtained from such methods can be stored, processed and analyzed as set forth herein.

Some SBS embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 A1; US 2009/0127589 A1; US 2010/0137143 A1; or US 2010/0282617 A1, each of which is incorporated herein by reference. Methods set forth herein for amplifying target nucleic acids using kinetic exclusion can be readily applied to substrates used for detecting protons. More specifically, methods set forth herein can be used to produce clonal populations of amplicons that are used to detect protons.

The above SBS methods can be advantageously carried out in multiplex formats such that multiple different target nucleic acids are manipulated simultaneously. In particular embodiments, different target nucleic acids can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the target nucleic acids can be in an array format. In an array format, the target nucleic acids can be typically bound to a surface in a spatially distinguishable manner. The target nucleic acids can be bound by direct covalent attachment, attachment to a bead or other particle or binding to a polymerase or other molecule that is attached to the surface. The array can include a single copy of a target nucleic acid at each site (also referred to as a feature) or multiple copies having the same sequence can be present at each site or feature. Multiple copies can be produced by amplification methods such as, bridge amplification or emulsion PCR as described in further detail below.

The methods set forth herein can use arrays having features at any of a variety of densities including, for example, at least about 10 features/cm2, 100 features/cm2, 500 features/cm2, 1,000 features/cm2, 5,000 features/cm2, 10,000 features/cm2, 50,000 features/cm2, 100,000 features/cm2, 1,000,000 features/cm2, 5,000,000 features/cm2, or higher.

An advantage of the methods set forth herein is that they provide for rapid and efficient detection of a plurality of target nucleic acid in parallel. Accordingly the present disclosure provides integrated systems capable of preparing and detecting nucleic acids using techniques known in the art such as those exemplified above. Thus, an integrated system of the present disclosure can include fluidic components capable of delivering amplification reagents and/or sequencing reagents to one or more immobilized DNA fragments, the system comprising components such as pumps, valves, reservoirs, fluidic lines and the like. A flow cell can be configured and/or used in an integrated system for detection of target nucleic acids. Exemplary flow cells are described, for example, in US 2010/0111768 A1 and US Ser. No. 13/273,666, each of which is incorporated herein by reference. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternatively, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeqTM platform (Illumina, Inc., San Diego, CA) and devices described in US Ser. No. 13/273,666, which is incorporated herein by reference.

As introduced above, nucleotides detected from a sample via methods such as the above sequencing methods may be termed as a read from the sample.

## Alternatives

Depending on the embodiment, certain acts, events, or functions of any of the algorithms described herein can be performed in a different sequence, can be added, merged, or left out altogether (e.g., not all described acts or events are necessary for the practice of the algorithm). Moreover, in certain embodiments, acts or events can be performed concurrently, e.g., through multi-threaded processing, interrupt processing, or multiple processors or processor cores or on other parallel architectures, rather than sequentially.

The various illustrative logical blocks, modules and algorithm steps described in connection with the embodiments disclosed herein can be implemented as electronic hardware, computer software or combinations of both. To clearly illustrate this interchangeability of hardware and software, various illustrative components, blocks, modules and steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing a departure from the scope of the disclosure.

The various illustrative logical blocks and modules described in connection with the embodiments disclosed herein can be implemented or performed by a machine, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be a controller, microcontroller, or state machine, combinations of the same, or the like. A processor can also be implemented as a combination of computing devices, e.g., a combination of a DSP and a microprocessor, a plurality of microprocessors, one or more microprocessors in conjunction with a DSP core, or any other such configuration.

The elements of a method, process, or algorithm described in connection with the embodiments disclosed herein can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM or any other form of computer-readable storage medium known in the art. A storage medium can be coupled to the processor such that the processor can read information from, and write information to, the storage medium. In the alternative, the storage medium can be integral to the processor. The processor and the storage medium can reside in an ASIC. The ASIC can reside in a user terminal. In the alternative, the processor and the storage medium can reside as discrete components in a user terminal.

Conditional language used herein, such as, among others, "can," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment. The terms "comprising," "including," "having," "involving," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some or all of the elements in the list.

Disjunctive language such as the phrase "at least one of X, Y or Z," unless specifically stated otherwise, is otherwise understood with the context as used in general to present that an item, term, etc., may be either X, Y or Z, or any combination thereof (e.g., X, Y and/or Z). Thus, such disjunctive language is not generally intended to, and should not, imply that certain embodiments require at least one of X, at least one of Y or at least one of Z to each be present.

Unless otherwise explicitly stated, articles such as "a" or "an" should generally be interpreted to include one or more described items. Accordingly, phrases such as "a device configured to" are intended to include one or more recited devices. Such one or more recited devices can also be collectively configured to carry out the stated recitations. For example, "a processor configured to carry out recitations A, B and C" can include a first processor configured to carry out recitation A working in conjunction with a second processor configured to carry out recitations B and C.

While the above detailed description has shown, described, and pointed out novel features as applied to various embodiments, it will be understood that various omissions, substitutions, and changes in the form and details of the devices or algorithms illustrated can be made without departing from the spirit of the disclosure. As will be recognized, certain embodiments described herein can be embodied within a form that does not provide all of the features and benefits set forth herein, as some features can be used or practiced separately from others. All changes which come within the meaning and range of equivalency of the claims are to be embraced within their scope.

The technologies from any example can be combined with the technologies described in any one or more of the other examples. In view of the many possible embodiments to which the principles of the disclosed technology may be applied, it should be recognized that the illustrated embodiments are examples of the disclosed technology and should not be taken as a limitation on the scope of the disclosed technology. Rather, the scope of the disclosed technology includes what is covered by the following claims. All that comes within the scope and spirit of the claims is therefore claimed.

**Claims**

1. A system for identifying candidate oncogenic splice variants from a patient comprising:

a memory;

at least one processor; and

at least one non-transitory computer readable medium containing instructions that, when executed by the at least one processor, cause the at least one processor to perform operations comprising:

determining one or more sample splice junctions from a plurality of RNA sequence reads from a single formalin-fixed paraffin-embedded (FFPE) tumor sample obtained from the patient;

retrieving a set of baseline splice junctions, the set of baseline splice junctions determined from a plurality of healthy RNA samples, wherein the plurality of healthy RNA samples comprises healthy RNA samples taken from a cross section of tissue types not obtained from the same biological object as the single FFPE tumor sample;

comparing the one or more sample splice junctions to the set of baseline splice junctions; and

identifying one or more filtered sample splice junctions, wherein the filtered sample splice junctions are sample splice junctions that do not overlap with the set of baseline splice junctions, wherein the one or more identified filtered sample splice junctions are candidate oncogenic events.

2. The system of claim 1, wherein the operations further comprise:

removing, from the identified filtered sample junctions, any junctions that overlap with non-cancerous splice variants determined from a splice graph that captures multiple alternate combinations of exons for a given gene.

3. The system of claim 1 or 2, further comprising outputting a list of candidate oncogenic events.

4. The system of any one of claims 1 to 3, wherein the plurality of healthy RNA samples comprises samples from one or more tissue types selected from the group consisting of: lung, adrenal gland, bladder, breast, ovary, liver, prostate, skin, and spleen, or wherein the plurality of healthy RNA samples comprises samples from donors across a range of ages.

5. The system of any one of claims 1-4, wherein the baseline splice junctions from the plurality of healthy RNA samples are determined prior to the determining the sample junctions from the single FFPE tumor sample.

6. The system of any one of claims 1-5, wherein the baseline junctions are from a same genomic region as the sample junctions, optionally, wherein the plurality of healthy RNA samples are from non-tumor tissue.

7. The system of any one of claims 1-6, wherein the sample splice junctions and the baseline splice junctions are both determined using a common test.

8. The system of any one of claims 1-7, wherein determining the one or more sample junctions comprises:

determining the plurality of RNA sequence reads from the single FFPE tumor sample;

retrieving, a DNA reference sequence aligned with the RNA sequence reads from the single FFPE tumor sample; and

determining one or more sample junctions as missing contiguous locations in the RNA read compared with the DNA reference.

9. A computer implemented method, comprising:

determining, using at least one processor, one or more sample splice junctions from a plurality of RNA sequence reads from a single formalin-fixed paraffin-embedded (FFPE) tumor sample obtained from a patient;

retrieving, by the at least one processor from a memory, a set of baseline splice junctions determined from a plurality of healthy RNA samples not obtained from the same biological object as the single FFPE tumor sample, wherein the plurality of healthy RNA samples comprises healthy RNA samples taken from a cross section of tissue types;

comparing the one or more sample splice junctions to the set of baseline splice junctions; and

identifying, by the at least one processor, one or more filtered sample splice junctions, wherein the filtered sample splice junctions are sample splice junctions that do not overlap with the baseline splice junctions, wherein the one or more of the identified filtered sample splice junctions are candidate oncogenic events.

10. The method of claim 9, further comprising: removing, from the identified filtered sample junctions, any junctions that overlap with non-cancerous splice variants determined from a splice graph that captures multiple alternate combinations of exons for a given gene.

11. The method of claim 9 or 10, further comprising outputting a list of candidate oncogenic events.

12. The method of any one of claims 9 to 11, further comprising:

determining, by the at least one processor, RNA reads from the single FFPE tumor sample;
retrieving, by the at least one processor from the memory, a DNA reference aligned with the RNA reads from the single FFPE tumor sample; and
determining, by the at least one processor, the sample junctions as missing contiguous locations in the RNA read compared with the DNA reference.

13. The method of any one of claims 9-12, wherein the plurality of healthy RNA samples comprises samples collected from one or more tissue types selected from the group consisting of: lung, adrenal gland, bladder, breast, ovary, liver prostate, skin, and spleen, or wherein the plurality of healthy RNA samples comprises donors across a range of ages.

14. The method of any one of claims 9-13, wherein the baseline splice junctions from the plurality of healthy RNA samples are determined prior to the determining the sample junctions from the single FFPE tumor sample.

15. The method of any one of claims 9-14, wherein the baseline junctions are from a same genomic region as the sample junctions, optionally wherein the plurality of healthy RNA samples are from non-tumor tissue.

FIG. 1

EP 4 787 379 A1

FIG. 2

EP 4 787 379 A1

Computing system    300

Processing unit
302

Network interface    304

Computer-readable medium drive
306

Input/output device interface
308

Memory    310

Interface Module
312

Operating system
314

Data processing module
316

FIG. 3

FIG. 4

500

Determine sample junctions
502

Determine baseline junctions
504

Determine filtered sample junctions
506

FIG. 5

502

Retrieve tumor sample reads from
single sample                612

Align
614

Determine sample junctions from
the single sample            616

Store the sample junctions
618

FIG. 6

504

Retrieve healthy sample reads from
cross sections of healthy tissue
samples                    712

Align
                          714

Determine baseline junctions from
the cross section of the healthy
tissue samples        716

Store the baseline junctions as a
baseline reference
                          718

FIG. 7

506

Retrieve the sample junctions
812

Retrieve the baseline junctions
814

Remove sample junctions with baseline junction overlap

824

Do the sample junctions overlap with the baseline junctions?
816

No

Yes

Collect as filtered sample junctions
818

Collect as overlap sample junctions
822

Verify junctions
820

Store filtered sample junctions
830

FIG. 8

900

Determine junction from first RNA
read from a sample   902

Determine additional junctions from
additional RNA reads from the
sample        904

Sufficient overlap
count?
906

No

Yes

Attribute as verified
908

FIG. 9

FIG. 10

FIG. 11

FIG. 12A

Exon 1-3 Splice Variant Reads

Non Splice Variant Reads

FIG. 12B

Europäisches
Patentamt

European
Patent Office

Office européen
des brevets

## EUROPEAN SEARCH REPORT

**Application Number**

EP 26 15 0173

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | QICHAO HUANG ET AL: "RNA-Seq Analyses Generate Comprehensive Transcriptomic Landscape and Reveal Complex Transcript Patterns in Hepatocellular Carcinoma", PLOS ONE, vol. 6, no. 10, 17 October 2011 (2011-10-17), page e26168, XP055461848, DOI: 10.1371/journal.pone.0026168 * abstract; p. 3, para. bridging left- to right-hand col.; p. 9, left-hand col., last para. * | 1-15 | INV. G16B20/20 |
| A | HEIDI DVINGE ET AL: "Widespread intron retention diversifies most cancer transcriptomes", GENOME MEDICINE, vol. 7, no. 1, 15 May 2015 (2015-05-15), XP055461877, DOI: 10.1186/s13073-015-0168-9 * p. 3, left-hand col., 1st para.; p. 3, right-hand col., 3rd para. * | 1-15 | |
| A | US 2006/199180 A1 (MACINA ROBERTO A [US] ET AL) 7 September 2006 (2006-09-07) * para. 14, 15, 449 * | 1-15 | **TECHNICAL FIELDS SEARCHED (IPC)** G16B |
| A | US 2002/081590 A1 (PENN SHARRON G [US] ET AL) 27 June 2002 (2002-06-27) * para. 175 * | 1-15 | |

-/--

The present search report has been drawn up for all claims

| Place of search | Date of completion of the search | Examiner |
|---|---|---|
| Munich | 22 June 2026 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 26 15 0173

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FENG MIN ET AL: "Survey of Programs Used to Detect Alternative Splicing Isoforms from Deep Sequencing Data In Silico", BIOMED RESEARCH INTERNATIONAL, vol. 2015, 1 January 2015 (2015-01-01), pages 1-9, XP055462042, ISSN: 2314-6133, DOI: 10.1155/2015/831352 * the whole document * ----- | 1-15 | |
| A | CN 105 989 246 A (BGI SHENZHEN) 5 October 2016 (2016-10-05) * the whole document * ----- | 1-15 | |

TECHNICAL FIELDS
SEARCHED     (IPC)

The present search report has been drawn up for all claims

| Place of search | Date of completion of the search | Examiner |
|---|---|---|
| Munich | 22 June 2026 | Ripaud, Leslie |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

 ....................................................................................

& : member of the same patent family, corresponding
 document

EPO FORM 1503 03.82 (P04C01)

page 2 of 2

## ANNEX TO THE EUROPEAN SEARCH REPORT
## ON EUROPEAN PATENT APPLICATION NO.

EP 26 15 0173

This annex lists the patent family members relating to the patent documents cited in the above-mentioned European search report.
The members are as contained in the European Patent Office EDP file on
The European Patent Office is in no way liable for these particulars which are merely given for the purpose of information.

22-06-2026

| Patent document cited in search report | Publication date | Patent family member(s) | | Publication date |
|---|---|---|---|---|
| US 2006199180 A1 | 07-09-2006 | AU | 2003258127 A1 | 23-02-2004 |
| | | US | 2006199180 A1 | 07-09-2006 |
| | | WO | 2004013311 A2 | 12-02-2004 |
| US 2002081590 A1 | 27-06-2002 | AU | 3087801 A | 14-08-2001 |
| | | AU | 3087901 A | 14-08-2001 |
| | | AU | 3088001 A | 14-08-2001 |
| | | AU | 3088101 A | 14-08-2001 |
| | | AU | 3088201 A | 14-08-2001 |
| | | AU | 3088301 A | 14-08-2001 |
| | | AU | 3275701 A | 14-08-2001 |
| | | AU | 3275801 A | 20-11-2001 |
| | | AU | 3275901 A | 14-08-2001 |
| | | AU | 3276001 A | 14-08-2001 |
| | | AU | 3311401 A | 14-08-2001 |
| | | AU | 3658901 A | 14-08-2001 |
| | | EP | 1290216 A2 | 12-03-2003 |
| | | EP | 1290217 A2 | 12-03-2003 |
| | | EP | 1292704 A2 | 19-03-2003 |
| | | EP | 1292705 A2 | 19-03-2003 |
| | | EP | 1309723 A2 | 14-05-2003 |
| | | EP | 1309724 A2 | 14-05-2003 |
| | | EP | 1309725 A2 | 14-05-2003 |
| | | EP | 1325149 A2 | 09-07-2003 |
| | | EP | 1325150 A2 | 09-07-2003 |
| | | EP | 1332224 A2 | 06-08-2003 |
| | | EP | 1341930 A2 | 10-09-2003 |
| | | GB | 2373500 A | 25-09-2002 |
| | | GB | 2374872 A | 30-10-2002 |
| | | GB | 2374929 A | 30-10-2002 |
| | | GB | 2375111 A | 06-11-2002 |
| | | GB | 2375539 A | 20-11-2002 |
| | | GB | 2376018 A | 04-12-2002 |
| | | GB | 2376237 A | 11-12-2002 |
| | | GB | 2378754 A | 19-02-2003 |
| | | GB | 2382814 A | 11-06-2003 |
| | | GB | 2383043 A | 18-06-2003 |
| | | GB | 2385053 A | 13-08-2003 |
| | | US | 2002081590 A1 | 27-06-2002 |
| | | WO | 0157251 A2 | 09-08-2001 |
| | | WO | 0157252 A2 | 09-08-2001 |
| | | WO | 0157270 A2 | 09-08-2001 |
| | | WO | 0157271 A2 | 09-08-2001 |
| | | WO | 0157272 A2 | 09-08-2001 |
| | | WO | 0157273 A2 | 09-08-2001 |
| | | WO | 0157274 A2 | 09-08-2001 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 26 15 0173

This annex lists the patent family members relating to the patent documents cited in  the above-mentioned European search report.
The members are as contained in the European Patent Office EDP file on
The European Patent Office is in no way liable for these particulars which are merely given for the purpose of information.

22-06-2026

| Patent document cited in search report | Publication date | Patent family member(s) | | Publication date |
|---|---|---|---|---|
| | | WO | 0157275 A2 | 09-08-2001 |
| | | WO | 0157276 A2 | 09-08-2001 |
| | | WO | 0157277 A2 | 09-08-2001 |
| | | WO | 0157278 A2 | 09-08-2001 |
| | | WO | 0186003 A2 | 15-11-2001 |
| CN 105989246 A | 05-10-2016 | NONE | | |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62447382
- US 6210891 B
- US 6258568 B
- US 6274320 B
- WO 04018497 A
- US 7057026 B
- WO 9106678 A
- WO 07123744 A
- US 7427673 B
- US 20070166705
- US 20060188901
- US 20060240439
- US 20060281109
- WO 05065814 A
- US 20050100900
- WO 06064199 A
- WO 07010251 A
- US 20120270305
- US 20130260372
- US 20130079232
- US 6969488 B
- US 6172218 B
- US 6306597 B
- US 7001792 B
- US 7329492 B
- US 7211414 B
- US 7315019 B
- US 7405281 B
- US 20080108082
- US 20090026082 A1
- US 20090127589 A1
- US 20100137143 A1
- US 20100282617 A1
- US 20100111768 A1
- US 273666

**Non-patent literature cited in the description**

- **JUNG, H.** ; **LEE, D.** ; **LEE, J.** ; **PARK, D** ; **KIM, Y. J.** ; **PARK, W.-Y.** ; **LEE, E.** Intron retention is a widespread mechanism of tumor-suppressor inactivation. *Nature Genetics*, 2015, vol. 47 (11), 1242-1248
- **BEN-HUR, V.** ; **DENICHENKO, P.** ; **SIEGFRIED, Z.** ; **MAIMON, A.** ; **KRAINER, A.** ; **DAVIDSON, B.** ; **KARNI, R.** S6K1 Alternative Splicing Modulates Its Oncogenic Activity and Regulates mTORC1. *Cell Reports*, 2013, vol. 3 (1), 103-115
- **DOBIN, DAVIS CA** ; **SCHLESINGER F** ; **DRENKOW J** ; **ZALESKI C** ; **JHA S** ; **BATUT P** ; **CHAISSON M** ; **GINGERAS TR.** STAR: ultrafast universal RNA-seq aligner. *Bioinformatics.*, 25 October 2012, vol. 29 (1), 15-21
- **RONAGHI, M.** ; **KARAMOHAMED, S** ; **PETTERSSON, B.** ; **UHLEN, M.** ; **NYREN, P.** Real-time DNA sequencing using detection of pyrophosphate release.. *Analytical Biochemistry*, 1996, vol. 242 (1), 84-9
- **RONAGHI, M.** Pyrosequencing sheds light on DNA sequencing.. *Genome Res.*, 2001, vol. 11 (1), 3-11
- **RONAGHI, M.** ; **UHLEN, M.** ; **NYREN, P.** A sequencing method based on real-time pyrophosphate.. *Science*, 1998, vol. 281 (5375), 363
- **METZKER**. *Genome Res*, 2005, vol. 15, 1767-1776
- **RUPAREL et al.** *Proc Natl Acad Sci USA*, 2005, vol. 102, 5932-7
- **DEAMER, D. W.** ; **AKESON, M.** Nanopores and nucleic acids: prospects for ultrarapid sequencing.. *Trends Biotechnol.*, 2000, vol. 18, 147-151
- **DEAMER, D.** ; **D. BRANTON**. Characterization of nucleic acids by nanopore analysis''.. *Acc. Chem. Res.*, 2002, vol. 35, 817-825
- **LI, J.** ; **M. GERSHOW** ; **D. STEIN** ; **E. BRANDIN** ; **J. A. GOLOVCHENKO**. DNA molecules and configurations in a solid-state nanopore microscope. *Nat. Mater.*, 2003, vol. 2, 611-615
- **SONI, G. V.** ; **MELLER**. A. Progress toward ultrafast DNA sequencing using solid-state nanopores.. *Clin. Chem.*, 2007, vol. 53, 1996-2001
- **HEALY, K.** Nanopore-based single-molecule DNA analysis.. *Nanomed.*, 2007, vol. 2, 459-481
- **COCKROFT, S. L.** ; **CHU, J.** ; **AMORIN, M.** ; **GHADIRI, M. R.** A single-molecule nanopore device detects DNA polymerase activity with single-nucleotide resolution.. *J. Am. Chem. Soc.*, 2008, vol. 130, 818-820
- **LEVENE, M. J. et al.** Zero-mode waveguides for single-molecule analysis at high concentrations.. *Science*, 2003, vol. 299, 682-686

- **LUNDQUIST, P. M. et al.** Parallel confocal detection of single molecules in real time.. *Opt. Lett.*, 2008, vol. 33, 1026-1028

- **KORLACH, J. et al.** Selective aluminum passivation for targeted immobilization of single DNA polymerase molecules in zero-mode waveguide nano structures.. *Proc. Natl. Acad. Sci. USA*, 2008, vol. 105, 1176-1181